# EUROPEAN PATENT APPLICATION

(11) **EP 1 331 269 A1**
(43) Date of publication of application: **30.07.2003**
(21) Application number: 01974681.7
(22) Date of filing: 04.10.2001
(51) Int. Cl.: C12N 15/12, C12N 1/21, C12N 9/48, C07K 16/40, C12Q 1/68, A61K 31/711, A61K 38/46, A61K 39/395, A61K 45/00, A61K 48/00, A61P 25/00, A61P 35/00, A61P 43/00, G01N 33/15, G01N 33/50, C12N 9/99

(54) **NOVEL PROTEIN, PROCESS FOR PRODUCING THE SAME AND USE THEREOF**

(30) Priority: 05.10.2000 JP 2000311715; 20.12.2000 JP 2000387771; 17.01.2001 JP 2001008897
(71) Applicant: Takeda Chemical Industries, Ltd., Osaka-shi, Osaka 541-0045 (JP)
(72) Inventor: ISHII, Takafumi, Tsukuba-shi, Ibaraki 305-0044 (JP); SUNAHARA, Eiji, Inashiki-gun, Ibaraki 300-0331 (JP)
(74) Representative: Rickard, Timothy Mark Adrian
(86) International application number: JP0108744
(87) International publication number: WO02029054

(57) **Abstract**

The present invention is intended to provide a novel protein, specifically to provide a protein comprising the same or substantially the same amino acid sequence as that represented by SEQ ID NO: 1 or a salt thereof; a DNA encoding the said protein; a medicament comprising the said protein or DNA; and a method of screening for a compound capable of enhancing or inhibiting the peptidase activity of the said protein.

The protein of the present invention and the DNA encoding the same can be used as a therapeutic and/or prophylactic agent for cancers or neurodegenerative diseases. The protein of the present invention and cells capable of expressing the gene of the said protein are useful as a material for screening for a compound or a salt thereof capable of enhancing or inhibiting the peptidase activity of the said protein.

SEQUENCE LISTING

<110> Takeda Chemical Industries, Ltd.

<120> Novel protein, process for producing the same and use thereof

<130> P2001-222PCT

<140> PCT/JP01/08744
<141> 2001-10-04

<150> JP 2000-311715
<151> 2000-10-05

<150> JP 2000-387771
<151> 2000-12-20

<150> JP 2001-8897
<151> 2001-01-17

<160> 24

<210> 1
<211> 795
<212> PRT
<213> Human

<400> 1

<210> 2
<211> 2385
<212> DNA
<213> Human

<400> 2
atgggagaga atgaagcaag tttacctaac acgtctttgc aaggtaaaaa gatggcctat    60
cagaaggtcc atgcagatca aagagctcca ggacactcac agtacttaga caatgatgac   120
cttcaagcca ctgcccttga cttagagtgg gacatggaga aggaactaga ggagtctggt   180
tttgaccaat tccagctaga cggtgctgag aatcagaacc tagggcattc agagactata   240
gacctcaatc ttgattccat tcaaccagca acttcaccca aaggaaggtt ccagagactt   300
caagaagaat ctgactacat tacccattat acacgatctg caccaaagag caatcgctgc   360
aacttttgcc acgtcttaaa aatactttgc acagccacca ttttatttat ttttggattt   420
ttgataggtt attatgtaca tacaaattgc ccttcagatg ctccatcttc aggaacagtt   480
gatcctcagt tatatcaaga gattctcaag acaatccagg cagaagatat taagaagtct   540
ttcagaaatt tggtacaact atataaaaat gaagatgaca cggaaatttc aaagaagatt   600
aagactcagt ggacctcttt gggcctagaa gatgtacagt ttgtaaatta ctctgtgctg   660
cttgatctgc caggcccttc tcccagcact gtgactctga gcagcagtgg tcaatgcttt   720
catcctaatg gccagccttg cagtgaagaa gccagaaaag atagcagcca agacctgctc   780

tattcatatg cagcctattc tgccaaagga actctcaagg ctgaagtcat cgatgtgagt 840
tatggaatgg cagatgattt aaaaaggatt aggaaaataa aaaacgtaac aaatcagatc 900
gcactcctga aattaggaaa attgccactg ctttataagc tttcctcatt ggaaaaggct 960
ggatttggag gtgttcttct gtatatcgat ccttgtgatt tgccaaagac tgtgaatcct 1020
agccatgata ccttcatggt gtcactgaat ccaggaggag acccttctac gcctggttac 1080
ccaagtgtcg atgaaagttt tagacaaagc cgatcaaacc tcacctctct attagtgcag 1140
cccatctctg catccctcgt tgcaaaactg atctcttcgc caaaagctag aaccaaaaat 1200
gaagcgtgta gctctctaga gcttccaaat aatgaaataa gagtcgtcag catgcaagtt 1260
cagacagtca caaaattgaa aacagttact aatgttgttg gatttgtaat gggcttgaca 1320
tctccagacc ggtatatcat agttggcagc catcatcaca ctgcacacag ttataatgga 1380
caagaatggg ccagtagtac tgcaataatc acagcgttta tccgtgcctt gatgtcaaaa 1440
gttaagagag ggtggagacc agaccgaact attgttttct gttcttgggg aggaacagct 1500
tttggcaata ttggctcata tgaatgggga gaggatttca agaaggttct tcagaaaaat 1560
gttgtggctt atattagcct ccacagtccc ataaggggga actctagtct gtatcctgta 1620
gcatcaccat ctcttcagca actggtagta gagaaaaata atttcaactg taccagaaga 1680
gcccagtgcc cagaaaccaa tatcagttct atacagatac aaggtgatgc tgattatttc 1740
atcaaccatc ttggagttcc catcgtgcag tttgcttacg aggacatcaa aacattagag 1800
ggtccaagtt ttctctccga ggcccgtttt tctacacgag caacaaaaat tgaagaaatg 1860
gatcgctctt tcaaccttca tgaaaccatt actaagctct caggagaagt gattttgcaa 1920
attgccaacg aacctgttct gccctttaat gcacttgata tagctttaga agttcaaaac 1980
aaccttaaag gtgatcaacc caacactcat caactgttag ccatggcgtt acgcctgcgg 2040
gagagtgctg aactttttca gtctgatgag atgcgacctg ctaatgatcc caaggagaga 2100
gcacccatcc gcatccggat gctgaatgac attctccaag acatggagaa aagctttctg 2160
gtaaagcagg caccaccagg tttttataga aacatcctct accaccttga tgaaaagaca 2220
agccggtttt caatacttat agaggcttgg gaacactgca aaccccttgc atcaaatgag 2280
acccttcaag aagccctgtc agaggtgttg aacagcatta attcagctca ggtttacttc 2340
aaagcaggac ttgatgtgtt caagagtgtc ttggatggga agaat 2385

<210> 3
<211> 795
<212> PRT
<213> Human

<400> 3
Met Gly Glu Asn Glu Ala Ser Leu Pro Asn Thr Ser Leu Gln Gly Lys
                5                   10                  15
Lys Met Ala Tyr Gln Lys Val His Ala Asp Gln Arg Ala Pro Gly His

            20                  25                  30
Ser Gln Tyr Leu Asp Asn Asp Asp Leu Gln Ala Thr Ala Leu Asp Leu
        35                  40                  45
Glu Trp Asp Met Glu Lys Glu Leu Glu Glu Ser Gly Phe Asp Gln Phe
    50                  55                  60
Gln Leu Asp Gly Ala Glu Asn Gln Asn Leu Gly His Ser Glu Thr Ile
65                  70                  75                  80
Asp Leu Asn Leu Asp Ser Ile Gln Pro Ala Thr Ser Pro Lys Gly Arg
                85                  90                  95
Phe Gln Arg Leu Gln Glu Glu Ser Asp Tyr Ile Thr His Tyr Thr Arg
            100                 105                 110
Ser Ala Pro Lys Ser Asn Arg Cys Asn Phe Cys His Val Leu Lys Ile
        115                 120                 125
Leu Cys Thr Ala Thr Ile Leu Phe Ile Phe Gly Ile Leu Ile Gly Tyr
    130                 135                 140
Tyr Val His Thr Asn Cys Pro Ser Asp Ala Pro Ser Ser Gly Thr Val
145                 150                 155                 160
Asp Pro Gln Leu Tyr Gln Glu Ile Leu Lys Thr Ile Gln Ala Glu Asp
                165                 170                 175
Ile Lys Lys Ser Phe Arg Asn Leu Val Gln Leu Tyr Lys Asn Glu Asp
            180                 185                 190
Asp Thr Glu Ile Ser Lys Lys Ile Lys Thr Gln Trp Thr Ser Leu Gly
        195                 200                 205
Leu Glu Asp Val Gln Phe Val Asn Tyr Ser Val Leu Leu Asp Leu Pro
    210                 215                 220
Gly Pro Ser Pro Ser Thr Val Thr Leu Ser Ser Ser Gly Gln Cys Phe
225                 230                 235                 240
His Pro Asn Gly Gln Pro Cys Ser Glu Glu Ala Arg Lys Asp Ser Ser
                245                 250                 255
Gln Asp Leu Leu Tyr Ser Tyr Ala Ala Tyr Ser Ala Lys Gly Thr Leu
            260                 265                 270
Lys Ala Glu Val Ile Asp Val Ser Tyr Gly Met Ala Asp Asp Leu Lys
        275                 280                 285
Arg Ile Arg Lys Ile Lys Asn Val Thr Asn Gln Ile Ala Leu Leu Lys
    290                 295                 300
Leu Gly Lys Leu Pro Leu Leu Tyr Lys Leu Ser Ser Leu Glu Lys Ala
305                 310                 315                 320

Gly Phe Gly Gly Val Leu Leu Tyr Ile Asp Pro Cys Asp Leu Pro Lys
                325                 330                 335
Thr Val Asn Pro Ser His Asp Thr Phe Met Val Ser Leu Asn Pro Gly
            340                 345                 350
Gly Asp Pro Ser Thr Pro Gly Tyr Pro Ser Val Asp Glu Ser Phe Arg
        355                 360                 365
Gln Ser Arg Ser Asn Leu Thr Ser Leu Leu Val Gln Pro Ile Ser Ala
    370                 375                 380
Ser Leu Val Ala Lys Leu Ile Ser Ser Pro Lys Ala Arg Thr Lys Asn
385                 390                 395                 400
Glu Ala Cys Ser Ser Leu Glu Leu Pro Asn Asn Glu Ile Arg Val Val
                405                 410                 415
Ser Met Gln Val Gln Thr Val Thr Lys Leu Lys Thr Val Thr Asn Val
            420                 425                 430
Val Gly Phe Val Met Gly Leu Thr Ser Pro Asp Arg Tyr Ile Ile Val
        435                 440                 445
Gly Ser His His His Thr Ala His Ser Tyr Asn Gly Gln Glu Trp Ala
    450                 455                 460
Ser Ser Thr Ala Ile Ile Thr Ala Phe Ile Arg Ala Leu Met Ser Lys
465                 470                 475                 480
Val Lys Arg Gly Trp Arg Pro Asp Arg Thr Ile Val Phe Cys Ser Trp
                485                 490                 495
Gly Gly Thr Ala Phe Gly Asn Ile Gly Ser Tyr Glu Trp Gly Glu Asp
            500                 505                 510
Phe Lys Lys Val Leu Gln Lys Asn Val Val Ala Tyr Ile Ser Leu His
        515                 520                 525
Ser Pro Ile Arg Gly Asn Ser Ser Leu Tyr Pro Val Ala Ser Pro Ser
    530                 535                 540
Leu Gln Gln Leu Val Val Glu Lys Asn Asn Phe Asn Cys Thr Arg Arg
545                 550                 555                 560
Ala Gln Cys Pro Glu Thr Asn Ile Ser Ser Ile Gln Ile Gln Gly Asp
                565                 570                 575
Ala Asp Tyr Phe Ile Asn His Leu Gly Val Pro Ile Val Gln Phe Ala
            580                 585                 590
Tyr Glu Asp Ile Lys Thr Leu Glu Gly Pro Ser Phe Leu Ser Glu Ala
        595                 600                 605
Arg Phe Ser Thr Arg Ala Thr Lys Ile Glu Glu Met Asp Arg Ser Phe

610                 615                 620
Asn Leu His Glu Thr Ile Thr Lys Leu Ser Gly Glu Val Ile Leu Gln
625                 630                 635                 640
Ile Ala Asn Glu Pro Val Leu Pro Phe Asn Ala Leu Asp Ile Ala Leu
                645                 650                 655
Glu Val Gln Asn Asn Leu Lys Gly Asp Gln Pro Asn Thr His Gln Leu
            660                 665                 670
Leu Ala Met Ala Ser Arg Leu Arg Glu Ser Ala Glu Leu Phe Gln Ser
        675                 680                 685
Asp Glu Met Arg Pro Ala Asn Asp Pro Lys Glu Arg Ala Pro Ile Arg
    690                 695                 700
Ile Arg Met Leu Asn Asp Ile Leu Gln Asp Met Glu Lys Ser Phe Leu
705                 710                 715                 720
Val Lys Gln Ala Pro Pro Gly Phe Tyr Arg Asn Ile Leu Tyr His Leu
                725                 730                 735
Asp Glu Lys Thr Ser Arg Phe Ser Ile Leu Ile Glu Ala Trp Glu His
            740                 745                 750
Cys Lys Pro Leu Ala Ser Asn Glu Thr Leu Gln Glu Ala Leu Ser Glu
        755                 760                 765
Val Leu Asn Ser Ile Asn Ser Ala Gln Val Tyr Phe Lys Ala Gly Leu
    770                 775                 780
Asp Val Phe Lys Ser Val Leu Asp Gly Lys Asn
785                 790                 795

<210> 4
<211> 2385
<212> DNA
<213> Human

<400> 4
atgggagaga atgaagcaag tttacctaac acgtctttgc aaggtaaaaa gatggcctat    60
cagaaggtcc atgcagatca aagagctcca ggacactcac agtacttaga caatgatgac    120
cttcaagcca ctgcccttga cttagagtgg gacatggaga aggaactaga ggagtctggt    180
tttgaccaat tccagctaga cggtgctgag aatcagaacc tagggcattc agagactata    240
gacctcaatc ttgattccat tcaaccagca acttcaccca aaggaaggtt ccagagactt    300
caagaagaat ctgactacat tacccattat acacgatctg caccaaagag caatcgctgc    360
aacttttgcc acgtcttaaa aatactttgc acagccacca ttttatttat ttttgggatt    420

ttgataggtt attatgtaca tacaaattgc ccttcagatg ctccatcttc aggaacagtt 480
gatcctcagt tatatcaaga gattctcaag acaatccagg cagaagatat taagaagtct 540
ttcagaaatt tggtacaact atataaaaat gaagatgaca cggaaatttc aaagaagatt 600
aagactcagt ggacctcttt gggcctagaa gatgtacagt ttgtaaatta ctctgtgctg 660
cttgatctgc caggcccttc tcccagcact gtgactctga gcagcagtgg tcaatgcttt 720
catcctaatg gccagccttg cagtgaagaa gccagaaaag atagcagcca agacctgctc 780
tattcatatg cagcctattc tgccaaagga actctcaagg ctgaagtcat cgatgtgagt 840
tatggaatgg cagatgattt aaaaaggatt aggaaaataa aaaacgtaac aaatcagatc 900
gcactcctga aattaggaaa attgccactg ctttataagc tttcctcatt ggaaaaggct 960
ggatttggag gtgttcttct gtatatcgat ccttgtgatt tgccaaagac tgtgaatcct 1020
agccatgata ccttcatggt gtcactgaat ccaggaggag acccttctac gcctggttac 1080
ccaagtgtcg atgaaagttt tagacaaagc cgatcaaacc tcacctctct attagtgcag 1140
cccatctctg catccctcgt tgcaaaactg atctcttcgc caaaagctag aaccaaaaat 1200
gaagcgtgta gctctctaga gcttccaaat aatgaaataa gagtcgtcag catgcaagtt 1260
cagacagtca caaaattgaa aacagttact aatgttgttg gatttgtaat gggcttgaca 1320
tctccagacc ggtatatcat agttggcagc catcatcaca ctgcacacag ttataatgga 1380
caagaatggg ccagtagtac tgcaataatc acagcgttta tccgtgcctt gatgtcaaaa 1440
gttaagagag ggtggagacc agaccgaact attgttttct gttcttgggg aggaacagct 1500
tttggcaata ttggctcata tgaatgggga gaggatttca agaaggttct tcagaaaaat 1560
gttgtggctt atattagcct ccacagtccc ataaggggga actctagtct gtatcctgta 1620
gcatcaccat ctcttcagca actggtagta gagaaaaata atttcaactg taccagaaga 1680
gcccagtgcc cagaaaccaa tatcagttct atacagatac aaggtgatgc tgattatttc 1740
atcaaccatc ttggagttcc catcgtgcag tttgcttacg aggacatcaa aacattagag 1800
ggtccaagtt ttctctccga ggcccgtttt tctacacgag caacaaaaat tgaagaaatg 1860
gatcgctctt tcaaccttca tgaaaccatt actaagctct caggagaagt gattttgcaa 1920
attgccaacg aacctgttct gccctttaat gcacttgata tagctttaga agttcaaaac 1980
aaccttaaag gtgatcaacc caacactcat caactgttag ccatggcgtc acgcctgcgg 2040
gagagtgctg aactttttca gtctgatgag atgcgacctg ctaatgatcc caaggagaga 2100
gcacccatcc gcatccggat gctgaatgac attctccaag acatggagaa aagctttctg 2160
gtaaagcagg caccaccagg tttttataga aacatcctct accaccttga tgaaaagaca 2220
agccggtttt caatacttat agaggcttgg gaacactgca aaccccttgc atcaaatgag 2280
acccttcaag aagccctgtc agaggtgttg aacagcatta attcagctca ggtttacttc 2340
aaagcaggac ttgatgtgtt caagagtgtc ttggatggga agaat 2385

<210> 5
<211> 21
<212> DNA

<213> Artificial Sequence

<220>
<223> Designed oligonucleotide primer to amplify DNA encoding novel prote
ins

<400> 5
ggagaccaga ccgaactatt g          21

<210> 6
<211> 23
<212> DNA
<213> Artificial Sequence

<220>
<223> Designed oligonucleotide primer to amplify DNA encoding novel prote
ins

<400> 6
tgaagagatg gtgatgctac agg          23

<210> 7
<211> 30
<212> DNA
<213> Artificial Sequence

<220>
<223> Designed oligonucleotide primer to amplify DNA encoding novel prote
ins

<400> 7
tgaagagatg gtgatgctac aggatacaga          30

<210> 8
<211> 26
<212> DNA
<213> Artificial Sequence

<220>
<223> Designed oligonucleotide primer to amplify DNA encoding novel prote
ins

<400> 8
gccaatattg ccaaaagctg ttcctc          26

<210> 9
<211> 30
<212> DNA
<213> Artificial Sequence

<220>
<223> Designed oligonucleotide primer to amplify DNA encoding novel prote
ins

<400> 9
gttggcagcc atcatcacac tgcacacagt          30

<210> 10
<211> 26
<212> DNA
<213> Artificial Sequence

<220>
<223> Designed oligonucleotide primer to amplify DNA encoding novel prote
ins

<400> 10
gaggaacagc ttttggcaat attggc          26

<210> 11
<211> 42
<212> DNA
<213> Artificial Sequence

<220>
<223> Designed oligonucleotide primer to amplify DNA encoding novel prote
ins

<400> 11
gctcgagatg ggagagaatg aagcaagttt acctaacacg tc        42

<210> 12
<211> 40
<212> DNA
<213> Artificial Sequence

<220>
<223> Designed oligonucleotide primer to amplify DNA encoding novel prote
ins

<400> 12
ggaattctca attcttccca tccaagacac tcttgaacac        40

<210> 13
<211> 795
<212> PRT
<213> Human

<400> 13

<210> 14
<211> 2385
<212> DNA
<213> Human

<400> 14
atgggagaga atgaagcaag tttacctaac acgtctttgc aaggtaaaaa gatggcctat 60
cagaaggtcc atgcagatca aagagctcca ggacactcac agtacttaga caatgatgac 120
cttcaagcca ctgcccttga cttagagtgg gacatggaga aggaactaga ggagtctggt 180
tttgaccaat tccagctaga cagtgctgag aatcagaacc tagggcattc agagactata 240
gacctcaatc ttgattccat tcaaccagca acttcaccca aaggaaggtt ccagagactt 300
caagaagaat ctgactacat tacccattat acacgatctg caccaaagag caatcgctgc 360
aacttttgcc acgtcttaaa aatgctttgc acagccacca ttttatttat ttttggattt 420
ttgataggtt attatgtaca tacaaattgc ccttcagatg ctccatcttc aggaacagtt 480
gatcctcagt tatatcaaga gattctcaag acaatccagg cagaagatat taagaagtct 540
ttcagaaatt tggtacaact atataaaaat gaagatgaca cggaaatttc aaagaagatt 600
aagactcagt ggacctcttt gggcctagaa gatgtacagt ttgtaaatta ctctgtgctg 660
cttgatctgc caggcccttc tcccagcact gtgactctga gcagcagtgg tcaatgcttt 720
catcctaatg gccagccttg cagtgaagaa gccagaaaag atagcagcca agacctgctc 780
tattcatatg cagcctattc tgccaaagga actctcaagg ctgaagtcat cgatgtgagt 840
tatggaatgg cagatgattt aaaaaggatt aggaaaataa aaaacgtaac aaatcagatc 900
gcactcctga aattaggaaa attgccactg ctttataagc tttcctcatt ggaaaaggct 960

ggatttggag gtgttcttct gtatatcgat ccttgtgatt tgccaaagac tgtgaatcct 1020
agccatgata ccttcatggt gtcactgaat ccaggaggag acccttctac gcctggttac 1080
ccaagtgtcg atgaaagttt tagacaaagc cgatcaaacc tcacctctct attagtgcag 1140
cccatctctg catccctcgt tgcaaaactg atctcttcgc caaaagctag aaccaaaaat 1200
gaagcgtgta gctctctaga gcttccaaat aatgaaataa gagtcgtcag catgcaagtt 1260
cagacagtca caaaattgaa aacagttact aatgttgttg gatttgtaat gggcttgaca 1320
tctccagacc ggtatatcat agttggcagc catcatcaca ctgcacacag ttataatgga 1380
caagaatggg ccagtagtac tgcaataatc acagcgttta tccgtgcctt gatgtcaaaa 1440
gttaagagag ggtggagacc agaccgaact attgttttct gttcttgggg aggaacagct 1500
tttggcaata ttggctcata tgaatgggga gaggatttca agaaggttct tcagaaaaat 1560
gttgtggctt atattagcct ccacagtccc ataaggggga actctagtct gtatcctgta 1620
gcatcaccat ctcttcagca actggtagta gagaaaaata atttcaactg taccagaaga 1680
gcccagtgcc cagaaaccaa tatcagttct atacagatac aaggtgatgc tgattatttc 1740
atcaaccatc ttggagttcc catcgtgcag tttgcttacg aggacatcaa aacattagag 1800
ggtccaagtt ttctctccga ggcccgtttt tctacacgag caacaaaaat tgaagaaatg 1860
gatcgctctt tcaaccttca tgaaaccatt actaagctct caggagaagt gattttgcaa 1920
attgccaacg aacctgttct gccctttaat gcacttgata tagctttaga agttcaaaac 1980
aaccttaaag gtgatcaacc caacactcat caactgttag ccatggcgtc acgcctgcgg 2040
gagagtgctg aactttttca gtctgatgag atgcgacctg ctaatgatcc caaggagaga 2100
gcacccatcc gcatccggat gctgaatgac attctccaag acatggagaa aagctttctg 2160
gtaaagcagg caccaccagg tttttataga aacatcctct accaccttga tgaaaagaca 2220
agccggtttt caatacttat agaggcttgg gaacactgca aaccccttgc atcaaatgag 2280
acccttcaag aagccctgtc agaggtgttg aacagcatta attcagctca ggtttacttc 2340
aaagcaggac ttgatgtgtt caagagtgtc ttggatggga agaat 2385

<210> 15
<211> 29
<212> DNA
<213> Artificial Sequence

<220>
<223> Primer for PCR

<400> 15
atgagtgaag aagccagaaa agatagcag 29

<210> 16

<211> 30
<212> DNA
<213> Artificial Sequence

<220>
<223> Primer for PCR

<400> 16
attcttccca tccaagacac tcttgaacac                    30

<210> 17
<211> 15
<212> PRT
<213> Artificial Sequence

<220>
<223> Synthetic peptide designed based on the amino acid sequence shown b
y SEQ ID NO: 1

<400> 17
Lys Leu Ile Ser Ser Pro Lys Ala Arg Thr Lys Asn Glu Ala Cys
                5                   10                  15

<210> 18
<211> 15
<212> PRT
<213> Artificial Sequence

<220>
<223> Synthetic peptide designed based on the amino acid sequence shown b
y SEQ ID NO: 1

<400> 18
Ser Asp Glu Met Arg Pro Ala Asn Asp Pro Lys Glu Arg Ala Cys
                5                   10                  15

<210> 19

<211> 30
<212> DNA
<213> Artificial Sequence

<220>
<223> Primer for PCR

<400> 19
atgggagaga atgaagcaag tttacctaac                                30

<210> 20
<211> 42
<212> DNA
<213> Artificial Sequence

<220>
<223> Primer for PCR

<400> 20
gctcgagatg ggagagaatg aagcaagttt acctaacacg tc                  42

<210> 21
<211> 40
<212> DNA
<213> Artificial Sequence

<220>
<223> Primer for PCR

<400> 21
ggaattctca attcttccca tccaagacac tcttgaacac                     40

<210> 22
<211> 21
<212> DNA
<213> Artificial Sequence

<220>
<223> Primer for TaqMan PCR

<400> 22
tcaaccatct tggagttccc a                                21

<210> 23
<211> 22
<212> DNA
<213> Artificial Sequence

<220>
<223> Primer for TaqMan PCR

<400> 23
cggagagaaa acttggaccc tc                               22

<210> 24
<211> 28
<212> DNA
<213> Artificial Sequence

<220>
<223> Probe for TaqMan PCR

<400> 24
cgtgcagttt gcttacgagg acatcaaa                         28

## Description

### TECHNICAL FIELD

The present invention relates to a novel peptidase protein (e.g. carboxypeptidase protein).

### BACKGROUND ART

It is known that a neuropeptide, N-acetyl-L-aspartyl-L-glutamate (NAAG) is present in both central nerves and peripheral nerves. NAAG is very abundant in brain and its content reaches a level of 10⁻³M order in some areas. NAAG is stored in synaptic vesicles and released by neuronal stimulation in a calcium-dependent manner (J. T. Coyle, Neurobiol. Dis. 4: 231-238, 1997). In central nervous system, NAAG is indicated to suppress the neurodegeneration in which N-methyl-D-aspartate (NMDA) or glutamic acid is involved, and it is shown to exhibit protection of neurons in a hippocampal slice sample or a cerebral cortex culture. It is thought that this effect is mediated by NAAG as an agonist of glutamic acid receptor type 2 (e.g. mGluR3) (V. Bruno et al., Neuroscience 85: 751-757, 1998). It is also indicated that NAAG may serve as a partial agonist of NMDA receptor (H. M. Valivullath et al., J. Neurochem. 63: 1714-1719, 1994).

It is reported that a human peptidase, in particular, a carboxypeptidase or N-acetylated α-linked acidic dipeptidase (NAALADase) is involved in degradation of NAAG (M. B. Robinson et al., J. Biol. Chem. 262: 14498-14506, 1987). This indicates that NAALADase may be involved deeply in neurodegenerative diseases caused by death of excitatory neuronal cells through glutamic acid release from NAAG. Moreover, since NAALADase activity is detected in a prostate-specific membrane antigen (PSMA) which is overexpressed in a prostate cancer (R. E. Carter et al., Proc. Natl. Acad. Sci. USA 93: 749-753, 1996), it draws attention in relation to cancers. PSMA has been expected to have an aminopeptidase activity and a transferrin receptor-like activity in addition to carboxypeptidase activity from similarity of the amino acid sequences (D. Mahadevan et al., Protein Sci. 8: 2546-2549, 1999). PSMA is now proven to have an aminopeptidase activity (M. N. Pangalos et al., J. Biol. Chem. 274: 8470-8483, 1999). An example of a protein having both receptor activity and peptidase activity is CD13/aminopeptidase N, which is a receptor of a tripeptide NGR that is involved in cell adhesion (R. Pasqualini et al., Cancer Res. 60: 722-727, 2000). Another example is Nicastrin, which is indicated to be involved in Alzheimer's disease and also has the similarity to the aminopeptidase and the transferrin receptor (R. Fagan et al., Trends Biochem. Sci. 26: 213-214, 2001). In this way, it is expected that a cell membrane-bound human peptidase protein may have a function as a receptor. There seem to be various ligands to respective peptidase proteins, for example, growth factors such as transferrin, β-amyloid preprotein, or physiologically active peptides.

A novel human peptidase protein makes it possible to develop a medical drug which is capable of regulating the activity of the protein and is useful for the prevention or treatment of various diseases associated with the activity, for example, central nervous and peripheral nervous diseases such as Alzheimer's disease, schizophrenia and the like. Further, since a novel human peptidase protein is predicted to increase much in human cancer tissues, the human peptidase protein may be used as a cancer vaccine by itself, and an antibody thereto or an immunotoxin having the antibody may be used for the treatment of cancers. Accordingly, in the technical field of the present invention, it has been desired to find a novel human-derived peptidase protein and to develop a method of producing the protein in a large amount.

### DISCLOSURE OF THE INVENTION

The present inventors intensively studied to resolve the foregoing object, and finally found a human-derived peptidase protein having a novel nucleic acid sequence. Based on this finding, the present inventors completed the present invention after further effort.

Thus, the present invention provides:
(1) A protein comprising the same or substantially the same amino acid sequence as that shown by SEQ ID NO: 1 or a salt thereof.
(2) The protein described in (1) comprising the amino acid sequence shown by SEQ ID NO: 1, NO: 3 or NO: 13 or a salt thereof.
(3) A partial peptide of the protein described in (1) or a salt thereof.
(4) A polynucleotide comprising a polynucleotide encoding the protein described in (1) or the partial peptide described in (3).
(5) The polynucleotide described in (4) which is a DNA.
(6) The DNA described in (5) comprising the nucleic acid sequence shown by SEQ ID NO: 2, NO: 4 or NO: 14.
(7) A recombinant vector comprising the polynucleotide described in (4).
(8) A transformant, which is transformed with the recombinant vector described in (7).
(9) A method of producing the protein described in (1) or a salt thereof, which comprises culturing the transformant described in (8) to produce and accumulate the protein described in (1); and recovering it.
(10) A pharmaceutical composition comprising the protein described in (1), the partial peptide described in (3), or a salt thereof.
(11) A pharmaceutical composition comprising the polynucleotide described in (4).
(12) The pharmaceutical composition described in (10) or (11), which is a therapeutic and/or prophylactic agent for cancers or neurological disorders.
(13) An antibody to the protein described in (1), the partial peptide described in (3) or a salt thereof.
(14) A diagnostic agent for cancers or neurological disorders, comprising the antibody described in (13).
(15) A complex comprising the antibody described in (13) and a toxin.
(16) A method of screening a compound or a salt thereof capable of enhancing or inhibiting the peptidase activity of the protein described in (1), the partial peptide described in (3) or a salt thereof, which comprises using the protein described in (1), the partial peptide described in (3) or a salt thereof.
(17) A kit for screening a compound or a salt thereof capable of enhancing or inhibiting the peptidase activity of the protein described in (1), the partial peptide described in (3) or a salt thereof, which comprises the protein described in (1), the partial peptide described in (3) or a salt thereof.
(18) A compound or a salt thereof capable of enhancing or inhibiting the peptidase activity of the protein described in (1), the partial peptide described in (3) or a salt thereof, which is obtained using the screening method described in (16) or the screening kit described in (17).
(19) A pharmaceutical composition comprising a compound or a salt thereof capable of enhancing or inhibiting the peptidase activity of the protein described in (1), the partial peptide described in (3) or a salt thereof, which is obtained using the screening method described in (16) or the screening kit described in (17).
(20) The pharmaceutical composition described in (19), which is a therapeutic and/or prophylactic agent for cancers or neurological disorders.
(21) A pharmaceutical composition comprising the antibody described in (13) or the complex described in (15).
(22) The pharmaceutical composition described in (21), which is a therapeutic and/or prophylactic agent for cancers.
(23) An antisense polynucleotide comprising a nucleic acid sequence which is complementary or substantially complementary to a polynucleotide encoding a protein having the same or substantially the same amino acid sequence as that shown by SEQ ID NO: 1 or a partial peptide thereof.
(24) A pharmaceutical composition comprising the antisense polynucleotide described in (23).
(25) The pharmaceutical composition described in (24), which is a therapeutic and/or prophylactic agent for cancers or neurological disorders.
(26) Use of the protein described in (1) or a salt thereof, the partial peptide described in (3) or a salt thereof, the polynucleotide described in (4), the antibody described in (13), the complex described in (15), the compound described in (18) or a salt thereof, or the antisense polynucleotide described in (23) for producing a pharmaceutical composition for the treatment and/or prevention of cancers or neurological disorders.
(27) A method of treating and/or preventing cancers or neurological disorders in a human or another mammal, which comprises administering to said human or mammal an therapeutically or prophylactically effective amount of the protein described in (1) or a salt thereof, the partial peptide described in (3) or a salt thereof, the polynucleotide described in (4), the antibody described in (13), the complex described in (15), the compound described in (18) or a salt thereof, or the antisense polynucleotide described in (23).
   Further, the present invention provides:
(28) A non-human mammal carrying a DNA encoding the protein of the present invention or a partial peptide thereof, or a mutant of the DNA.
(29) The non-human mammal described in (28), which is a rodent.
(30) The non-human mammal described in (29), wherein the rodent is a mouse or rat.
(31) A recombinant vector comprising the DNA encoding the protein of the present invention or a partial peptide thereof, or a mutant of the DNA, and being capable of expressing the DNA or the mutant in a mammal.
(32) An embryonic stem cell of a non-human mammal, wherein the DNA encoding the protein of the present invention or a partial peptide thereof is inactivated.
(33) The embryonic stem cell described in (32), wherein the DNA is inactivated by introducing a reporter gene (e.g. β-galactosidase gene derived from E. coli) into the DNA.
(34) The embryonic stem cell described in (32), which is neomycin-resistant.
(35) The embryonic stem cell described in (32), wherein the non-human mammal is a rodent.
(36) The embryonic stem cell described in (35), wherein the rodent is a mouse,
(37) A non-human mammal, wherein the DNA encoding the protein of the present invention or a partial peptide thereof is inactivated, and is insufficiently expressed.
(38) The non-human mammal described in (37), wherein the DNA encoding the protein of the present invention or a partial peptide thereof is inactivated by introducing a reporter gene (e.g. β-galactosidase gene derived from E. coli) into the DNA, and the reporter gene is expressed under the control of the promoter for the DNA.
(39) The non-human mammal described in (37), which is a rodent.
(40) The non-human mammal described in (39), wherein the rodent is a mouse.

### BEST MODES FOR CARRYING OUT THE INVENTION

A protein comprising the same or substantially the same amino acid sequence as that shown by SEQ ID NO: 1 (hereinafter, referred to as the protein of the present invention) may be derived from any cells of warm-blooded animals (e.g. human, guinea pig, rat, mouse, chicken, rabbit, swine, sheep, bovine, monkey, etc.) such as hepatocyte, splenocyte, neuronal cell, glial cell, pancreatic β-cell, bone marrow cell, mesangial cell, Langerhans' cell, epidermic cell, epithelial cell, endothelial cell, fibroblast, fibrocyte, myocyte, adipocyte, immunocyte (e.g. macrophage, T cell, B cell, natural killer cell, mast cell, neutrophil, basophil, eosinophil, monocyte), megakaryocyte, synovial cell, chondrocyte, bone cell, osteoblast, osteoclast, mammary gland cell, hepatocyte, or interstitial cell; or the corresponding precursor cells, stem cells, cancer cells, etc.; or any tissues where such cells are present, such as brain or any brain regions (e.g. olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g. large and small intestines), blood vessel (e.g. aorta), heart, thymus, spleen, submandibular gland, peripheral blood, prostate, testis, ovary, placenta, uterus, bone, joint, skeletal muscle, etc.; or blood cells or cultured cells thereof (e.g. MEL, M1, CTLL-2, HT-2, WEHI-3, HL-60, JOSK-1, K562, ML-1, MOLT-3, MOLT-4, MOLT-10, CCRF-CEM, TALL-1, Jurkat, CCRT-HSB-2, KE-37, SKW-3, HUT-78, HUT-102, H9, U937, THP-1, HEL, JK-1, CMK, KO-812, MEG-01, etc.). The protein may also be synthesized.

The amino acid sequence which is substantially the same as that shown by SEQ ID NO: 1 includes an amino acid sequence having about 50% or more, preferably about 60% or more, more preferably about 70% or more, further preferably about 80% or more, especially preferably about 90% or more, or most preferably about 95% or more homology to the amino acid sequence shown by SEQ ID NO: 1.

The protein of the present invention comprising substantially the same amino acid sequence as that shown by SEQ ID NO: 1 preferably includes a protein having substantially the same amino acid sequence as that shown by SEQ ID NO: 1 and also having substantially the same activity (e.g. peptidase activity) as that of the protein having the amino acid sequence shown by SEQ ID NO: 1.

The protein of the present invention comprising substantially the same amino acid sequence as that shown by SEQ ID NO: 1 includes a protein comprising the amino acid sequence shown by SEQ ID NO: 3, a protein comprising the amino acid sequence shown by SEQ ID NO: 13, and the like.

As used herein, the peptidase activity, especially the carboxypeptidase activity means the activity to release the carboxy-terminal amino acid from an α- or γ-bonding acidic peptide as a substrate. Such a substrate peptide may be a naturally occurring substrate such as N-acetyl-L-aspartyl-L-glutamate (NAAG), and also a synthetic peptide such as folyl-poly-γ-glutamate and L-γ-glutamylglutamate.

The carboxypeptidase activity can be determined according to a well-known method, and also to the screening method described below.

Further, the protein of the present invention includes so-called muteins of the protein comprising the amino acid sequence shown by SEQ ID NO: 1, for example, (i) wherein one or more (1 to about 30, preferably 1 to about 10, more preferably several (1 to 5)) amino acids are deleted from the amino acid sequence shown by SEQ ID NO: 1; (ii) wherein one or more (1 to about 30, preferably 1 to about 10, more preferably several (1 to 5)) amino acids are added to the amino acid sequence shown by SEQ ID NO: 1; (iii) wherein one or more (1 to about 30, preferably 1 to about 10, more preferably several (1 to 5)) amino acids are inserted into the amino acid sequence shown by SEQ ID NO: 1; (iv) wherein one or more (1 to about 30, preferably 1 to about 10, more preferably several (1 to 5)) amino acids in the amino acid sequence shown by SEQ ID NO: 1 are substituted with other amino acids; or (v) wherein the amino acid sequence shown by SEQ ID NO: 1 are modified in combination with the above.

The protein of the present invention may be preferably a protein comprising the amino acid sequence shown by SEQ ID NO: 1, NO: 3 or NO: 13.

In this specification, proteins are represented in accordance with the conventional way of describing peptides so as to place the N-terminal (amino terminal) on the left side and the C-terminal (carboxyl terminal) on the right side. In the protein of the present invention including the protein comprising the amino acid sequence shown by SEQ ID NO: 1, the C-terminal may be in the form of carboxyl group (-COOH), carboxylate (-COO'), amide (-CONH₂) or ester (-COOR).

Examples of R in the ester include a C₁₋₆ alkyl group such as methyl, ethyl, n-propyl, isopropyl, n-butyl; a C₃₋₈ cycloalkyl group such as cyclopentyl, cyclohexyl; a C₆₋₁₂ aryl group such as phenyl, α-naphthyl; a C₇₋₁₄ aralkyl group such as a phenyl-C₁₋₂-alkyl group (e.g. benzyl, phenethyl), an α-naphthyl-C₁₋₂-alkyl group (e.g. α-naphthylmethyl); and the like. In addition, pivaloyloxymethyl and the like, which are used widely as an ester for oral administration, may also be used.

When the protein of the present invention has a carboxyl group (or a carboxylate) at a position other than the C-terminal, it may be amidated or esterified. Such an amide or ester is also included within the protein of the present invention. The ester in this case may be the same as described above with respect to the ester of C-terminal.

Furthermore, the protein of the present invention includes variants thereof, wherein the amino group at the N-terminal methionine residue is protected with a protecting group (for example, a C₁₋₆ acyl group such as a C₁₋₆ alkanoyl group, e.g. formyl group, acetyl group); those wherein a glutamyl group at the N-terminal, which is formed due to cleavage in vivo, is pyroglutaminated; those wherein a substituent (e.g. -OH, -SH, amino group, imidazole group, indole group, guanidino group) on the side chain of an amino acid in the molecule is protected with a suitable protecting group (for example, a C₁₋₆ acyl group such as a C₁₋₆ alkanoyl group, e.g. formyl group, acetyl group); or conjugated proteins such as glycoproteins bound to sugar chains.

The partial peptide of the protein of the present invention may be any peptides which are derived from the protein of the present invention and preferably have the same activity as that of the protein of the present invention (e.g. peptidase activity). The partial peptide of the present invention may have an amino acid sequence of, for example, at least 20, preferably at least 50, more preferably at least 70, even more preferably at least 100, most preferably 200 amino acids of the constitutional amino acid sequence of the protein of the present invention, and may have the peptidase activity.

The partial peptide of the present invention can be used as an antigen for producing an antibody, and thus it may not necessary have the peptidase activity.

The salt of the protein or the partial peptide of the present invention includes salts with physiologically acceptable acids (e.g. inorganic acids, organic acids) or bases (e.g. alkali metal salts). Especially, a physiologically acceptable acid addition salt is preferred. Examples of the salt include a salt with an inorganic acid (e.g. hydrochloric acid, phosphoric acid, hydrobromic acid, sulfuric acid) or with an organic acid (e.g. acetic acid, formic acid, propionic acid, fumaric acid, maleic acid, succinic acid, tartaric acid, citric acid, malic acid, oxalic acid, benzoic acid, methanesulfonic acid, benzenesulfonic acid).

The protein of the present invention or a salt thereof can be produced by a well-known purification method from cells or tissues of the above-mentioned warm-blooded animals, or by culturing a transformant comprising a polynucleotide (e.g. DNA) encoding the protein as described below. Furthermore, the protein or a salt thereof can also be produced by a peptide synthesis method as described below.

In order to produce the protein or a salt thereof from tissues or cells of warm-blooded animals, the tissues or cells are homogenized, extracted with an acid or the like, and then the thus obtained extract is subjected to a combination of chromatography techniques such as reverse phase chromatography, ion exchange chromatography and the like to isolate and purify the protein.

To synthesize the protein of the present invention, a partial peptide, a salt, or an amide thereof, commercially available resins that are used for protein synthesis may be usually used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmehtylphenyl acetamidomethyl resin, polyacrylamide resin, 4-(2',4'-dimethoxyphenylhydroxymethyl) phenoxy resin, 4-(2',4'-dimethoxyphenyl-Fmoc-aminoethyl) phenoxy resin, etc. Using these resins, amino acids in which α-amino groups and functional groups on the side chains are appropriately protected are condensed on the resin in the order of the sequence of the objective protein according to various condensation methods publicly known in the art. At the end of the reaction, the protein is cut out from the resin and at the same time, the protecting groups are removed. Then, intramolecular disulfide bond-forming reaction is performed in a highly diluted solution to obtain the objective protein or an amide thereof.

For condensation of the protected amino acids described above, a variety of activating reagents for protein synthesis may be used, and carbodiimides are particularly preferable. Examples of such carbodiimides include DCC, N,N'-diisopropylcarbodiimide, N-ethyl-N'-(3-dimethylaminoprolyl)carbodiimide. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by applying the thus activated protected amino acids to the resin.

Solvents suitable to activate the protected amino acids or condense with the resin may be chosen from solvents known to be usable for protein condensation reactions. Examples of such solvents are acid amides such as N,N-dimethylformamide, N,N-dimethylacetamide, N-methylpyrrolidone; halogenated hydrocarbons such as methylene chloride, chloroform; alcohols such as trifluoroethanol; sulfoxides such as dimethylsulfoxide; ethers such as pyridine, dioxane, tetrahydrofuran; nitriles such as acetonitrile, propionitrile; esters such as methyl acetate, ethyl acetate; and appropriate mixtures of these solvents. The reaction temperature is appropriately chosen from the range known to be applicable to the protein binding reaction and is usually selected in the range of approximately -20°C to 50°C. The activated amino acid derivatives are used generally in an excess of 1.5 to 4 times. The condensation is examined using the ninhydrin reaction test. When the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole to avoid an adverse affect on the subsequent reaction.

Examples of groups to protect amino groups of the starting compounds include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc.

A carboxyl group can be protected by, for example, alkyl esterification (e.g. esterification with a linear, branched or cyclic alkyl such as methyl, ethyl, propyl, butyl, t-butyl, cyclopentyl, cyclohexyl, cycloheptyl, cyclooctyl, 2-adamantyl), aralkyl esterification (e.g. esterification with benzyl, 4-nitrobenzyl, 4-methoxybenzyl, 4-chlorobenzyl, benzhydryl), phenacyl esterification; benzyloxycarbonyl hydrazidation, t-butoxycarbonyl hydrazidation, trityl hydrazidation.

The hydroxyl group of serine can be protected by, for example, esterification or etherification. Examples of groups suitable for the esterification include a lower alkanoyl group such as acetyl group, an aroyl group such as benzoyl group, and a group derived from carbonic acid such as benzyloxycarbonyl group, ethoxycarbonyl group. Examples of a group suitable for the etherification include benzyl group, tetrahydropyranyl group, t-butyl group.

Examples of groups to protect the phenolic hydroxyl group of tyrosine include Bzl, Cl₂-Bzl, 2-nitrobenzyl, Br-Z, t-butyl.

Examples of groups to protect the imidazole moiety of histidine include Tos, 4-methoxy-2,3,6-trimethylbenzenesulfonyl, DNP, benzyloxymethyl, Bum, Boc, Trt, Fmoc.

Examples of the activated carboxyl group in the starting material include the corresponding acid anhydride, azide, activated ester (ester with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)). Examples of the activated amino group in the starting material include a phosphoric amide.

To eliminate (remove) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethane-sulfonic acid or trifluoroacetic acid, or a mixture solution of these acids; a treatment with a base such as diisopropylethylamine, triethylamine, piperidine or piperazine; and reduction with sodium in liquid ammonia. The elimination of protecting groups by the acid treatment described above is carried out generally at a temperature of approximately -20°C to 40°C. In the acid treatment, it is effective to add a cation scavenger such as anisole, phenol, thioanisole, m-cresol, p-cresol, dimethylsulfide, 1,4-butanedithiol or 1,2-ethanedithiol. Furthermore, 2,4-dinitrophenyl group used as the protecting group for the imidazole of histidine is removed by a treatment with thiophenol. Formyl group used as the protecting group of the indole of tryptophan is eliminated by the aforesaid acid treatment in the presence of 1,2-ethanedithiol or 1,4-butanedithiol, as well as by a treatment with an alkali such as a dilute sodium hydroxide solution or dilute ammonia.

Protection of functional groups that should not be involved in the reaction of the starting materials, protecting groups for this, elimination of the protecting groups, and activation of functional groups involved in the reaction may be appropriately selected from well-known groups and well-known means.

In another method for obtaining an amide of the protein, the α-carboxyl group of the carboxy terminal amino acid is first protected by amidation; the peptide (protein) chain is then extended from the amino group side to a desired length. Thereafter, a protein in which only the protecting group of the N-terminal α-amino group in the peptide chain has been eliminated from the protein and a protein in which only the protecting group of the C-terminal carboxyl group has been eliminated are prepared. The two proteins are condensed by mixture in the solvent described above. The details of the condensation reaction are the same as described above. After the protected protein obtained by the condensation is purified, all the protecting groups are eliminated by the method described above to give the desired crude protein. This crude protein is purified by various known purification means. Lyophilization of the major fraction gives the desired amide form of the protein.

To prepare the esterified protein, for example, the α-carboxyl group of the carboxy terminal amino acid is condensed with a desired alcohol to prepare the amino acid ester, which is then subjected to a process similar to that for the preparation of the amidated protein above to give the desired ester form of the protein.

The partial peptide of the present invention or a salt thereof can be produced by well-known peptide synthesis methods, or by cleaving the protein of the present invention with an appropriate peptidase. For the peptide synthesis methods, for example, either solid phase synthesis or liquid phase synthesis may be used. That is, a partial peptide or an amino acid that can constitute the partial peptide of the present invention are condensed with the remaining part. When the product contains protecting groups, these protecting groups are removed to give the desired peptide. Well-known methods for condensation and elimination of the protecting groups are described in 1) - 5) below.
1) M. Bodanszky & M.A. Ondetti: Peptide Synthesis, Interscience Publishers, New York (1966)
2) Schroeder & Luebke: The Peptide, Academic Press, New York (1965)
3) Nobuo Izumiya, et al.: *Peptide Gosei-no-Kiso to Jikken* (Basics and experiments of peptide synthesis), published by Maruzen Co. (1975)
4) Haruaki Yajima & Shunpei Sakakibara: *Seikagaku Jikken Koza* (Biochemical Experiment) 1, *Tanpakushitsu no Kagaku* (Chemistry of Proteins) IV, 205 (1977)
5) Haruaki Yajima, ed.: *Zoku lyakuhin no Kaihatsu* (A sequel to Development of Pharmaceuticals), Vol. 14, Peptide Synthesis, published by Hirokawa Shoten

After the reaction is completed, the partial peptide of the present invention may be purified and isolated by a combination of conventional purification methods such as solvent extraction, distillation, column chromatography, liquid chromatography and recrystallization. When the partial peptide thus obtained is in a free form, the peptide can be converted into an appropriate salt according to a well-known method or a variant thereof. On the contrary, when the protein is obtained in a salt form, it can be converted into a free form or another salt form by a well-known method or a variant thereof.

The polynucleotide encoding the protein of the present invention includes any polynucleotide comprising a nucleotide sequence encoding the protein of the present invention as described above, which is preferably a DNA. The DNA may be derived from any of genomic DNA, genomic DNA library, cDNA derived from the cells and tissues described above, cDNA library derived from the cells and tissues described above, and synthetic DNA.

The DNA may also be directly amplified as a cDNA by reverse transcriptase polymerase chain reaction (hereinafter abbreviated as RT-PCR) using total RNAs or an mRNA fraction prepared from the cells and tissues described above.

Specifically, the DNA encoding the protein of the present invention includes (i) a DNA which comprises the nucleic acid sequence shown by SEQ ID NO: 2, or a DNA which is hybridizable to the DNA comprising the nucleic acid sequence shown by SEQ ID NO: 2, and encodes a protein having substantially the same activity (e.g. peptidase activity) as that of the protein of the present invention; (ii) a DNA which comprises the nucleic acid sequence shown by SEQ ID NO: 4, or a DNA which is hybridizable to the DNA comprising the nucleic acid sequence shown by SEQ ID NO: 4, and encodes a protein having substantially the same activity (e.g. peptidase activity) as that of the protein of the present invention; (iii) a DNA which comprises the nucleic acid sequence shown by SEQ ID NO: 14, or a DNA which is hybridizable to the DNA comprising the nucleic acid sequence shown by SEQ ID NO: 14, and encodes a protein having substantially the same activity (e.g. peptidase activity) as that of the protein of the present invention.

Examples of the DNA hybridizable to the nucleic acid sequence shown by SEQ ID NO: 2, NO: 4 or NO: 14 include a DNA comprising a nucleic acid sequence having at least about 70 % homology, preferably at least about 80 % homology, more preferably at least about 90 % homology, and most preferably at least about 95 % homology to the nucleic acid sequence shown by SEQ ID NO: 2, NO: 4 or NO: 14.

The hybridization can be carried out by a well-known method or a variant thereof, for example, the method described in Molecular Cloning, 2nd (J. Sambrook et al., Cold Spring Harbor Lab. Press, 1989). A commercially available library may also be used according to the instructions of the attached manufacturer's protocol. More preferably, the hybridization can be carried out under highly stringent condition.

The highly stringent condition as used herein is, for example, a sodium concentration of about 19 mM to 40 mM, preferably about 19 mM to 20 mM; and a temperature of about 50°C to 70°C, preferably about 60°C to 65°C. In particular, the hybridization condition having a sodium concentration of about 19 mM and a temperature of about 65°C is most preferred.

More specifically, the DNA encoding the protein comprising the amino acid sequence shown by SEQ ID NO: 1 includes a DNA comprising the nucleic acid sequence shown by SEQ ID NO: 2. The DNA encoding the protein comprising the amino acid sequence shown by SEQ ID NO: 3 includes a DNA comprising the nucleic acid sequence shown by SEQ ID NO: 4. The DNA encoding the protein comprising the amino acid sequence shown by SEQ ID NO: 13 includes a DNA comprising the nucleic acid sequence shown by SEQ ID NO: 14.

The DNA encoding the partial peptide of the present invention may be any DNA comprising the nucleotide sequence encoding the partial peptide of the present invention as described above, and may be derived from any of genomic DNA, genomic DNA library, cDNA derived from the cells and tissues described above, cDNA library derived from the cells and tissues described above, and synthetic DNA.

Specifically, the DNA encoding the partial peptide of the present invention includes (i) a DNA having a part of a DNA which has the nucleic acid sequence shown by SEQ ID NO: 2, or having a part of a DNA which is hybridizable to the DNA comprising the nucleic acid sequence shown by SEQ ID NO: 2, and encodes a protein having substantially the same activity as that of the protein of the present invention; (ii) a DNA having a part of a DNA which has the nucleic acid sequence shown by SEQ ID NO: 4, or having a part of a DNA which is hybridizable to the DNA comprising the nucleic acid sequence shown by SEQ ID NO: 4, and encodes a protein having substantially the same activity as that of the protein of the present invention; (iii) a DNA having a part of a DNA which has the nucleic acid sequence shown by SEQ ID NO: 14, or having a part of a DNA which is hybridizable to the DNA comprising the nucleic acid sequence shown by SEQ ID NO: 14, and encodes a protein having substantially the same activity as that of the protein of the present invention.

The hybridization method and the highly stringent condition are as described above.

For cloning of the DNA that completely encodes the protein of the present invention or the partial peptide thereof (hereinafter generically referred to simply as the protein of the present invention in the description of the DNA cloning and expression), the DNA may be amplified by PCR using synthetic DNA primers having a part of the nucleic acid sequence encoding the protein of the present invention. Alternatively, the DNA inserted into an appropriate vector can be selected by hybridization with a labeled DNA fragment or synthetic DNA that encodes a part or entire region of the protein of the present invention. The hybridization can be carried out, for example, according to the method described in Molecular Cloning, 2nd, J. Sambrook et al., Cold Spring Harbor Lab. Press, 1989. The hybridization may also be performed using a commercially available library in accordance with the protocol described in the attached instruction.

The exchange of the nucleic acid sequence of the DNA can be effected by PCR or a well-known method such as ODA-LA PCR method, Gapped duplex method or Kunkel method, or a variant thereof, using a well-known kit available as Mutan™-super Express Km (Takara Shuzo) or Mutan™-K (Takara Shuzo).

The thus cloned DNA encoding the protein of the present invention can be used depending upon purpose, as it is or if desired, after digestion with a restriction enzyme or after addition of a linker thereto. The DNA may have ATG as a translation initiation codon at the 5'-end and may further has TAA, TGA or TAG as a translation termination codon at the 3'-end. These translation initiation codon and termination codon may also be added using an appropriate synthetic DNA adapter.

The expression vector for the protein of the present invention can be produced, for example, by (a) excising the desired DNA fragment from the DNA, for example, cDNA encoding the protein of the present invention, and then (b) ligating the DNA fragment downstream of a promoter in an appropriate expression vector.

5 Examples of the vector include plasmids derived form E. coli (e.g., pBR322, pBR325, pUC12, pUC13), plasmids derived from Bacillus subtilis (e.g., pUB110, pTP5, pC194), plasmids derived from yeast (e.g., pSH19, pSH15), bacteriophages such as λ-phage, animal viruses such as retrovirus, vaccinia virus, baculovirus as well as pA1-11, pXT1, pRc/CMV, pRc/RSV, pcDNAI/Neo.

The promoter used in the present invention may be any promoter suitable for a host to be used for gene expression. When using animal cells as the host, the promoter includes SRα promoter, SV40 promoter, LTR promoter, CMV (cytomegalovirus) promoter, HSV-TK promoter. Among them, CMV promoter or SRα promoter is preferably used.

When using bacteria of the genus Escherichia as the host, the preferred promoter includes trp promoter, lac promoter, recA promoter, λP_{L} promoter, lpp promoter, T7 promoter. When using bacteria of the genus Bacillus as the host, the preferred promoter includes SPO1 promoter, SPO2 promoter and penP promoter. When using yeast as the host, the preferred promoter includes PHO5 promoter, PGK promoter, GAP promoter and ADH promoter. When using insect cells as the host, the preferred promoter includes polyhedrin prompter and P10 promoter.

In addition to the foregoing examples, the expression vector may further optionally contain an enhancer, a splicing signal, a poly A addition signal, a selection marker, SV40 replication origin (hereinafter abbreviated as SV40ori), and the like. The selection marker includes dihydrofolate reductase (hereinafter abbreviated as dhfr) gene [methotrexate (MTX)-resistant], ampicillin resistant gene (hereinafter abbreviated as Amp^{r}), neomycin-resistant gene (hereinafter abbreviated as Neo^{r}, G418-resistant). In particular, when using dhfr gene as the selection marker in dhfr-deficient CHO cells, the target gene can also be selected in a thymidine-free medium.

If necessary and desired, a signal sequence suitable for a host is added to the N-terminus of the protein of the present invention. Examples of the signal sequence that can be used include Pho A signal sequence, OmpA signal sequence in case of using bacteria of the genus Escherichia as the host; α-amylase signal sequence, subtilisin signal sequence in case of using bacteria of the genus Bacillus as the host; MFα signal sequence, SUC2 signal sequence in case of using yeast as the host; and insulin signal sequence, α-interferon signal sequence, antibody molecule signal sequence in case of using animal cells as the host, respectively.

By using the vector comprising the DNA encoding the protein of the present invention thus constructed, a transformant can be produced.

For example, bacteria of the genus Escherichia, bacteria of the genus Bacillus, yeasts, insect cells, insects and animal cells may be used as the host.

Examples of bacteria of the genus Escherichia include Escherichia coli K12 DH1 (Proc. Natl. Acad. Sci. U.S.A., 60, 160 (1968)), JM103 (Nucleic Acids Research, 9, 309 (1981)), JA221 (Journal of Molecular Biology, 120, 517 (1978)), HB101 (Journal of Molecular Biology, 41, 459 (1969)), C600 (Genetics, 39, 440 (1954)).

Examples of bacteria of the genus Bacillus include Bacillus subtilis MI114 (Gene, 24, 255 (1983)), 207-21 (Journal of Biochemistry, 95, 87 (1984)).

Examples of yeasts include Saccharomyces cereviseae AH22, AH22R⁻, NA87-11A, DKD-5D, 20B-12, Schizosaccharomyces pombe NCYC1913, NCYC2036, Pichia pastoris KM71.

Examples of insect cells include, for the virus AcNPV, Spodoptera frugiperda cells (Sf cells), MG1 cells derived from mid-intestine of Trichoplusia ni, High Five™ cells derived from egg of Trichoplusia ni, cells derived from Mamestra brassicae, cells derived from Estigmena acrea, etc.; and for the virus BmNPV, Bombyx mori N cells (BmN cells), etc. are used. Examples of the Sf cell which can be used are Sf9 cells (ATCC CRL1711) and Sf21 cells (both cells are described in Vaughn, J. L. et al., In Vivo, 13, 213-217 (1977).

Examples of insects include a larva of Bombyx mori (Maeda, et al., Nature, 315, 592 (1985)).

Examples of animal cells include monkey COS-7 cells, Vero cells, Chinese hamster cells CHO (hereinafter referred to as CHO cells), dhfr gene- deficient Chinese hamster cells CHO (hereinafter referred to as CHO (dhfr⁻) cell), mouse L cells, mouse AtT-20 cells, mouse myeloma cells, rat GH3 cells, human FL cells.

Bacteria of the genus Escherichia can be transformed, for example, by the method described in Proc. Natl. Acad. Sci. U.S.A., 69, 2110 (1972) or Gene, 17, 107 (1982).

Bacteria of the genus Bacillus can be transformed, for example, by the method described in Molecular & General Genetics, 168, 111 (1979).

Yeast can be transformed, for example, by the method described in Methods in Enzymology, 194, 182-187 (1991) or Proc. Natl. Acad. Sci. USA, 75, 1929 (1978).

Insect cells or insects can be transformed, for example, according to the method described in Bio/Technology, 6, 47-55(1988).

Animal cells can be transformed, for example, according to the method described in *Saibo Kogaku*, extra issue 8, *Shin Saibo Kogaku Jikken Protocol,* 263-267 (1995) (published by Shujunsha) or Virology, 52, 456 (1973).

In this way, a transformant, which is transformed with the expression vector comprising the DNA encoding the protein of the present invention, can be obtained.

When using bacteria of the genus Escherichia or the genus Bacillus as the host, the transformant can be appropriately incubated in a liquid medium which contains materials required for growth of the transformant such as carbon sources, nitrogen sources, inorganic materials, and so on. Examples of the carbon sources include glucose, dextrin, soluble starch, sucrose. Examples of the nitrogen sources include inorganic or organic materials such as ammonium salts, nitrate salts, corn steep liquor, peptone, casein, meat extract, soybean cake, potato extract. Examples of the inorganic materials are calcium chloride, sodium dihydrogenphosphate, magnesium chloride. In addition, yeast extract, vitamins, growth promoting factors, etc. may also be added to the medium. Preferable pH of the medium is from about 5 to 8.

A preferred medium for culture of bacteria of the genus Escherichia is M9 medium supplemented with glucose and Casamino acids (Miller, Journal of Experiments in Molecular Genetics, 431-433, Cold Spring Harbor Laboratory, New York, 1972). If necessary, a chemical such as 3β-indolylacrylic acid can be added to the medium thereby to enhance the efficiency of the promoter.

When using bacteria of the genus Escherichia as the host, the transformant is usually cultivated at about 15 to 43°C for about 3 to 24 hours. If necessary, the culture may be aerated or agitated.

When using bacteria of the genus Bacillus as the host, the transformant is cultivated generally at about 30 to 40°C for about 6 to 24 hours. If necessary, the culture can be aerated or agitated.

When using yeasts as the host, the transformant is cultivated, for example, in Burkholder's minimal medium (Bostian, K. L. et al., Proc. Natl. Acad. Sci. U.S.A., 77, 4505 (1980)) or SD medium supplemented with 0.5% Casamino acids (Bitter, G. A. et al., Proc. Natl. Acad. Sci. U.S.A., 81, 5330 (1984)). Preferably, pH of the medium is adjusted to about 5 to 8. In general, the transformant is cultivated at about 20 to 35°C for about 24 to 72 hours. If necessary, the culture can be aerated or agitated.

When using insect cells or insects as the host, the transformant is cultivated in, for example, Grace's Insect Medium (Grace, T. C. C., Nature, 195, 788 (1962)) to which an appropriate additive such as immobilized 10% bovine serum is optionally added.
Preferably, pH of the medium is adjusted to about 6.2 to 6.4. Normally, the transformant is cultivated at about 27°C for about 3 to 5 days and, if necessary, the culture can be aerated or agitated.

When using animal cells as the host, the transformant is cultivated in, for example, MEM medium containing about 5 to 20% fetal bovine serum (Science, 122, 501 (1952)), DMEM medium (Virology, 8, 396 (1959)), RPMI 1640 medium (The Journal of the American Medical Association, 199, 519 (1967)), 199 medium (Proceeding of the Society for the Biological Medicine, 73, 1 (1950)). Preferable pH of the medium is about 6 to 8. The transformant is usually cultivated at about 30 to 40°C for about 15 to 60 hours and, if necessary, the culture can be aerated or agitated.

As described above, the protein of the present invention can be produced inside the transformant cell, in the cell membrane, or outside the cell.

The protein of the present invention can be separated and purified from the culture described above by the following procedures.

To extract the protein of the present invention from the cultured bacteria or cells, the bacteria or cells are collected by a well-known method after cultivation, and suspended in an appropriate buffer. The bacteria or cells are then disrupted by a well-known method such as ultrasonication, a treatment with lysozyme and/or freeze-thaw process, followed by centrifugation, filtration, etc. to obtain the crude protein extract. The buffer used for this procedure may contain a protein modifier such as urea or guanidine hydrochloride, or a surfactant such as Triton X-100™. When the protein is secreted into the culture medium, after completion of the cultivation, the supernatant can be separated from the bacteria or cells to collect the supernatant by a well-known method.

The protein contained in the supernatant or the extract thus obtained can be purified by an appropriate combination of well-known methods for separation and purification. Such known methods for separation and purification include a method utilizing difference in solubility such as salting out, solvent precipitation; a method utilizing mainly difference in molecular weight such as dialysis, ultrafiltration, gel filtration, SDS-polyacrylamide gel electrophoresis; a method utilizing difference in electric charge such as ion exchange chromatography; a method utilizing difference in specific affinity such as affinity chromatography; a method utilizing difference in hydrophobicity such as reverse phase high peuormance liquid chromatography; a method utilizing difference in isoelectric point such as isoelectrofocusing electrophoresis; and the like.

When the protein thus obtained is in a free form, it can be converted into a salt by a well-known method or a variant thereof. On the other hand, when the protein is obtained in a salt form, it can be converted into the free form or another salt form by a well-known method or a variant thereof.

The protein of the present invention, produced by the recombinant, can be modified, before or after the purification, with an appropriate protein-modifying enzyme, or can be partially deleted. Examples of the protein-modifying enzyme include trypsin, chymotrypsin, arginyl endopeptidase, protein kinase, glycosidase and the like.

The thus produced protein of the present invention can be detected by an enzyme immunoassay using a specific antibody, and the activity thereof can be determined by measuring the amount of glutamic acid released due to degradation of N-acetyl-L-aspartyl-L-glutamate (NAAG).

The antibody to the protein of the present invention, the partial peptide, or the salt thereof may be any polyclonal antibody or monoclonal antibody, which is capable of recognizing the protein of the present invention, the partial peptide, or the salt thereof.

The antibody to the protein of the present invention, the partial peptide, or the salt thereof (hereinafter sometimes generically referred to as the antibody of the present invention) can be produced according to a well-known method for producing an antibody or antiserum, using as an antigen the protein of the present invention.

### [Preparation of monoclonal antibody]

### (a) Preparation of monoclonal antibody-producing cells

The protein of the present invention is administered to warm-blooded animals either alone or together with carriers or diluents to the site where the production of antibody is possible by the administration. In order to potentiate the antibody productivity upon the administration, complete Freund's adjuvants or incomplete Freund's adjuvants may be administered. The administration is usually carried out once in every two to six weeks and 2 to 10 times in total. Examples of the applicable warm-blooded animals are monkeys, rabbits, dogs, guinea pigs, mice, rats, sheep, goats and chicken, with mice and rats being preferred.

To prepare monoclonal antibody-producing cells, warm-blooded animals, e.g., mice, are immunized with an antigen. The mouse whose antibody titer is noted is selected, and then its spleen or lymph node is collected after 2 to 5 days from the final immunization. Antibody-producing cells contained therein are fused with myeloma cells of a homogeneous or heterogeneous animal to give a monoclonal antibody-producing hybridoma. Measurement of the antibody titer in antisera may be carried out, for example, by reacting a labeled form of the protein, which will be described later, with the antiserum, followed by assaying the binding activity of the labeling agent bound to the antibody. The fusion may be operated, for example, by the known Koehler and Milstein method (Nature, 256, 495, 1975). Examples of the fusion accelerator are polyethylene glycol (PEG), Sendai virus, etc., of which PEG is preferably employed.

Examples of the myeloma cells are ones derived from warm-blooded animals such as NS-1, P3U1, SP2/0, AP-1, etc. In particular, P3U1 is preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by incubating at about 20 to 40°C, preferably at about 30 to 37°C for about 1 to 10 minutes, an efficient cell fusion can be carried out.

Various methods can be used for screening of a monoclonal antibody-producing hybridoma. Examples of such methods include a method which comprises adding the supernatant of hybridoma to a solid phase (e.g., microplate) adsorbed with the protein as an antigen directly or together with a carrier, adding an anti-immunoglobulin antibody (when mouse cells are used for the cell fusion, anti-mouse immunoglobulin antibody is used) labeled with a radioactive substance or an enzyme, or Protein-A, and detecting the monoclonal antibody bound to the solid phase; and a method which comprises adding the supernatant of hybridoma to a solid phase adsorbed with an anti-immunoglobulin antibody or Protein-A, adding the protein labeled with a radioactive substance or an enzyme and detecting the monoclonal antibody bound to the solid phase.

The monoclonal antibody can be selected by a well-known method or a variant thereof. In general, the selection can be effected in a medium for animal cells supplemented with HAT (hypoxanthine, aminopterin and thymidine). Any selection and growth medium can be employed as far as the hybridoma can grow therein. For example, RPMI 1640 medium containing 1 to 20 %, preferably 10 to 20 % fetal bovine serum, GIT medium (Wako Pure Chemical Industries, Ltd.) containing 1 to 10 % fetal bovine serum, a serum free medium for cultivation of a hybridoma (SFM-101, Nissui Seiyaku Co., Ltd.) and the like can be used for the selection and growth medium. The cultivation is carried out generally at 20 to 40°C, preferably at about 37°C, for 5 days to 3 weeks, preferably 1 to 2 weeks. The cultivation can be conducted normally in 5 % CO₂. The antibody titer of the culture supernatant of hybridoma can be determined as in the assay for the antibody titer in antiserum described above.

### (b) Purification of monoclonal antibody

Separation and purification of a monoclonal antibody can be carried out by a well-known method, for example, a separation and purification method for immunoglobulins [e.g. salting-out, alcohol precipitation, isoelectric point precipitation, electrophoresis, adsorption and desorption with ion exchangers (e.g. DEAE), ultracentrifugation, gel filtration, or a specific purification method which comprises collecting only an antibody with an activated adsorbent such as an antigen-binding solid phase, Protein A, Protein G, etc. and dissociating the binding to obtain the antibody].

### [Preparation of polyclonal antibody]

The polyclonal antibody of the present invention can be produced by a well-known method or a variant thereof. For example, a complex of immunogen (the protein of the present invention as an antigen) and a carrier protein is prepared, and a warm-blooded animal is immunized with the complex in a manner similar to the method described above for the preparation of a monoclonal antibody. The product containing the antibody to the protein of the present invention is collected from the immunized animal, followed by separation and purification of the antibody.

For the complex of an immunogen and a carrier protein used to immunize a warm-blooded animal, the type of carrier protein and the mixing ratio of a carrier to the hapten may be any type and in any ratio, as long as the antibody is efficiently produced to the immunized hapten crosslinked to the carrier. For example, bovine serum albumin, bovine thyroglobulins, or keyhole limpet hemocyanin is coupled to the hapten in a carrier-to-hapten weight ratio of approximately 0.1 to 20, preferably about 1 to 5.

A variety of condensing agents can be used for the coupling of a carrier to hapten. Glutaraldehyde, carbodiimide, maleimide-activated ester, or activated ester reagents containing thiol group or dithiopyridyl group, etc. are used for the coupling.

The condensation product is administered to warm-blooded animals either alone or together with carriers or diluents to the site where the antibody can be produce by the administration. In order to potentiate the antibody productivity upon the administration, complete Freund's adjuvant or incomplete Freund's adjuvant may be administered. The administration is usually made once approximately in every 2 to 6 weeks and about 3 to 10 times in total.

The polyclonal antibody can be collected from the blood, ascites, etc., preferably from the blood, of a warm-blooded animal immunized by the method described above.

The polyclonal antibody titer in antiserum can be assayed by the same procedure as that for the determination of antibody titer in antiserum described above. The separation and purification of the polyclonal antibody can be carried out according to the separation and purification method for immunoglobulins, which is applied to the separation and purification of a monoclonal antibody as described above.

In the following, specifically described are utilities of the protein of the present invention, the partial peptide or the salt thereof (hereinafter sometimes referred to as the protein of the present invention), utilities of the polynucleotide encoding the protein of the present invention or the partial peptide thereof (hereinafter sometimes referred to as the DNA of the present invention), utilities of the antibody to the protein of the present invention, the partial peptide or the salt thereof (hereinafter sometimes referred to as the antibody of the present invention), and utilities of the antisense polynucleotide (e.g. antisense DNA).

### [1] A prophylactic and/or therapeutic agent for various diseases associated with the protein of the present invention

In the nervous system, the protein of the present invention is involved in the control of degradation of the neuropeptide, N-acetyl-L-aspartyl-L-glutamate (NAAG) and the neurotransmission based on the glutamic acid release from NAAG Accordingly, when the polynucleotide encoding the protein of the present invention has a mutation or a deletion, or when the expression amount of the protein is decreased, various neurological diseases such as dementia and defect of memory are generated.

Thus, the protein of the present invention and the polynucleotide of the present invention can be used as a medicine such as a prophylactic and/or therapeutic agent for various neurological diseases such as dementia and defect of memory.

When the neurotransmitter, glutamic acid is not sufficiently supplied nor normally expressed in a patient due to decrease or deficiency in the protein of the present invention in vivo, the protein can be made to work sufficiently or normally in the patient (a) by administering the polynucleotide of the present invention to the patient to express the protein of the present invention in vivo, (b) by incorporating the polynucleotide of the present invention into a cell to express the protein of the present invention, and then transplanting the cell to the patient, or (c) by administering the protein of the present invention to the patient.

When the polynucleotide of the present invention is used as the prophylactic/therapeutic agent described above, the polynucleotide can be administered to a human or another warm-blooded animal in a conventional manner by itself or by inserting the polynucleotide into an appropriate vector such as retrovirus vector, adenovirus vector, adenovirus-associated virus vector. The polynucleotide of the present invention may also be administered as it is, or with a physiologically acceptable carrier such as an adjuvant to assist its uptake, with the gene gun or a catheter such as a catheter with a hydrogel.

When the protein of the present invention is used as the therapeutic/prophylactic agent, the protein is advantageously used at a purity of at least 90 %, preferably at least 95 %, more preferably at least 98 % and most preferably at least 99 %.

The protein of the present invention can be used orally, for example, in the form of tablets which may be sugar coated if necessary, capsules, elixirs, microcapsules etc., or parenterally in the form of injectable preparations such as a sterile solution and a suspension in water or with other pharmaceutically acceptable liquid. These preparations can be manufactured by mixing the protein of the present invention with a physiologically acceptable carrier, a flavoring agent, an excipient, a vehicle, an antiseptic agent, a stabilizer, a binder, etc. in a unit dosage form required for a generally accepted pharmaceutical preparation. The active ingredient in the preparation is adjusted appropriately within the specified range given.

Additives miscible in a tablet, a capsule, etc. include a binder such as gelatin, corn starch, tragacanth and gum arabic; an excipient such as crystalline cellulose; a swelling agent such as corn starch, gelatin and alginic acid; a lubricant such as magnesium stearate; a sweetening agent such as sucrose, lactose and saccharin; and a flavoring agent such as peppermint, akamono oil and cherry. When the unit dosage is in the form of a capsule, liquid carriers such as oils and fats may further be used together with the additives described above. A sterile composition for injection may be formulated by conventional procedures used to make pharmaceutical compositions, e.g., by dissolving or suspending the active ingredients in a vehicle such as water for injection with a naturally occurring vegetable oil such as sesame oil and coconut oil, etc. to prepare the pharmaceutical composition.

Examples of an aqueous medium for injection include a physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol or the like), a polyalcohol (e.g., propylene glycol and polyethylene glycol), a nonionic surfactant (e.g., polysorbate 80™ and HCO-50), etc. Examples of the oily medium include sesame oil and soybean oil, which may also be used in combination with a dissolution aid such as benzyl benzoate and benzyl alcohol. The composition may further contain a buffer (e.g., phosphate buffer, sodium acetate buffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus-prepared liquid injection is normally filled in an appropriate ampoule.

The vector into which the polynucleotide of the present invention is inserted may also be formulated as a pharmaceutical preparation in a manner similar to the procedures above. Such a preparation is generally used parenterally.

Since the thus obtained pharmaceutical preparation is safe and low toxic, it can be administered to a warm-blooded animal (e.g. human, rat, mouse, guinea pig, rabbit, bird, sheep, swine, bovine, horse, cat, dog, monkey, chimpanzee).

The dose of the protein of the present invention varies depending on a target disease, a subject to be administered, a route for administration, etc. For example, in oral administration of the protein to an adult (60 kg body weight) for the treatment of nervous diseases, the daily dose is normally about 0.1 to 100 mg, preferably about 1.0 to 50 mg, and more preferably about 1.0 to 20 mg of the protein. In parenteral administration, the single dose also varies depending on a subject to be administered, a target disease, etc., and it is advantageous to administer the protein intravenously at a daily dose of about 0.01 to 30 mg, preferably about 0.1 to 20 mg, and more preferably about 0.1 to 10 mg to an adult (60 kg body weight) for the treatment of nervous diseases. For other animal species, the corresponding dose as converted per 60 kg body weight can be administered.

On the other hand, the protein of the present invention is over-expressed in cancers such as a prostate cancer, and thus in another embodiment, it can be used as a cancer vaccine to activate the immune system in a patient with a cancer.

For example, so-called, the adoptive immunotherapy can preferably be used, which comprises culturing a strong antigen-presenting cell (e.g. a dendritic cell) in the presence of the protein of the present invention to make the cell phagocytize the protein, and returning back the cell into the patient. The dendritic cell, returned back into the body, can induce and/or activate a cytotoxic T cell specific to a cancer antigen whereby to kill a cancer cell.

Thus, the protein of the present invention can be administered to a warm-blooded animal including a mammal (e.g. a human, monkey, mouse, rat, rabbit, pig) as a vaccine preparation to prevent or treat, for example, a cancer.

The vaccine preparation usually contains the protein of the present invention and a physiologically acceptable carrier. Such a carrier includes a liquid carrier such as water, saline (e.g. physiological saline), buffer (e.g. phosphate buffer), alcohol (e.g. ethanol). In general, the protein of the present invention is dissolved or suspended in a physiologically acceptable carrier. Alternatively, the protein of the present invention and the physiologically acceptable carrier may be separately prepared, and then mixed at use.

The vaccine preparation may contain, for example, an adjuvant such as aluminum hydroxide gel, serum albumin, a preservative such as thimerosal, glucose, a soothing agent such as benzyl alcohol, in addition to the protein and the carrier. Further, the vaccine preparation may also contain a cytokine (an interleukin such as interleukin-2, an interferon such as interferon-γ) to enhance the production of the antibody to the protein of the present invention.

When used as a vaccine preparation, the protein of the present invention may be in an active form, or in a denatured form to enhance the antigenicity of the protein. The protein can be denatured usually by heating or treating with a protein- denaturing agent (e.g. formalin, guanidine hydrochloride, urea).

The vaccine preparation as described above is produced according to a conventional method of producing a vaccine preparation.

The thus obtained vaccine preparation is low toxic and may be usually administered in an injectable form subcutaneously, intracutaneously, intramuscularly, or locally into or near a mass of cancer cells.

The dose of the protein of the present invention varies depending on a target disease, a subject to be administered, a route for administration, etc. For example, for subcutaneous administration of the protein to a cancer patient (weighing 60 kg) in an injectable form, the single dose is normally about 0.1 to 300 mg, preferably about 100 to 300 mg. The administration of the vaccine preparation may be carried out once, or 2 to 4 times in total in every 2 weeks to 6 months to increase the production of the antibody.

### [2] Screening of a pharmaceutical compound candidate for diseases

The protein of the present invention, a partial peptide thereof, or a salt thereof (hereinafter sometimes generically referred to as the protein of the present invention) is over-expressed in a cancer tissue, and thus a compound or a salt thereof capable of inhibiting the function of the protein can be used as a medicine such as a therapeutic and/or prophylactic agent for a cancer (e.g. cancers of stomach, large intestine, rectum, colon, lung, breast, uterine cervix, prostate, ovary, liver, pancreas; chronic lymphatic leukemia, chronic myelocytic leukemia, malignant melanoma, multiple myeloma).

Further, the protein of the present invention is involved in the degradation of NAAG capable of preventing neurodegeneration, and thus a compound or a salt thereof capable of inhibiting the function of the protein can be also used as a medicine such as a therapeutic and/or prophylactic agent for neurological diseases (e.g. neurodegenerative diseases such as Alzheimer's disease, schizophrenia, Parkinson's disease, peripheral nerve disease. Huntington's disease, acute brain damage, multiple sclerosis, ALS (amyotrophic lateral sclerosis), peripheral nerve damage, brain ischaemia).

On the other hand, the protein of the present invention is involved in the control of degradation of NAAG and the neurotransmission based on the glutamic acid release, and thus a compound or a salt thereof capable of enhancing the function of the protein (e.g. peptidase activity) can be also used as a medicine such as a therapeutic and/or prophylactic agent for various neurological diseases such as dementia and defect of memory.

Accordingly, the protein of the present invention or the polynucleotide of the present invention is useful as a reagent for screening of a compound or a salt thereof capable of enhancing or inhibiting the function of the protein.

Thus, the present invention provides a method for screening of a compound or a salt thereof capable of enhancing or inhibiting the function of the protein (e.g. peptidase activity), which comprises using the protein of the present invention, a partial peptide thereof, or a salt thereof (hereinafter sometimes generically referred to as the protein of the present invention).

Specifically, the present invention provides:
(1) a method of screening for a compound or a salt thereof having the activity of enhancing or inhibiting the peptidase activity of the protein of the present invention, which comprises:
   culturing cells capable of expressing the gene of the protein in the presence of a test compound; and
   measuring the amount of mRNA encoding the protein using the DNA encoding the protein or its cDNA or its partial DNA;
      and more specifically,
(2) a method of screening for a compound or a salt thereof having the activity of enhancing or inhibiting the peptidase activity of the protein of the present invention, which comprises:
   comparing (i) the amount of mRNA of the protein when culturing the cells capable of expressing the gene of the protein and (ii) the amount of mRNA of the protein when culturing the cells capable of expressing the gene of the protein in the presence of a test compound.

Examples of the cells capable of expressing the gene of the protein of the present invention include cells from known warm-blooded animals as described above (preferably prostate cancer cells, etc.) and animal cells transformed with the gene of the protein of the present invention. The animal cells transformed with the gene of the protein of the present invention can be produced by the aforementioned methods.

The culture of the cells capable of expressing the gene of the protein of the present invention can be conducted in a similar manner to known methods for animal cell culture. For example, as a culture medium for the animal cells, used are MEM medium (Science, 122, 501 (1952)), DMEM medium (Virology, 8, 396 (1959)), RPMI 1640 medium (The Journal of the American Medical Association, 199, 519 (1967)), or 199 medium (Proceeding of the Society for the Biological Medicine, 73, 1 (1950)), all of which contains about 5 to 20 % fetal bovine serum. Preferable pH of the medium is about 6 to 8. The culture is usually conducted at about 30 to 40°C for about 15 to 60 hours and, if necessary, the culture may be aerated or agitated.

The hybridization for comparison of expression amount of the mRNA can be carried out by a well-known methods or a variant thereof, for example, the method described in Molecular Cloning, 2nd (J. Sambrook et al., Cold Spring Harbor Lab. Press, 1989).

Specifically, quantification of the mRNA encoding the protein of the present invention is carried out by contacting RNAs, extracted from the cells according to a known method, with the genetic DNA encoding the protein or its cDNA or its partial DNA; and measuring an amount of mRNA bound to the genetic DNA encoding the protein or its cDNA. The amount of mRNA bound to the cDNA of the genetic DNA encoding the protein is easily determined using, for example, a radioisotope- or dye-labeled form of the cDNA of the genetic DNA encoding the protein or its partial DNA. Examples of the radioisotope include ³²P, ³H, and the like, and examples of the dye include fluorescein, FAM (PE Biosystems), JOE (PE Biosystems), TAMAR (PE Biosystems), ROX (PE Biosystems), Cy5 (Amersham), Cy3 (Amersham), and the like.

It is also possible to quantifying the mRNA encoding the protein of the present invention by preparing cDNAs by reverse trasncriptase from RNAs extracted from the cells; carrying out a PCR of the prepared cDNAs using the genetic DNA encoding the protein or its cDNA or its partial DNA as primers; and measuring an amount of the amplified cDNA product.

The aforementioned cDNA of the genetic DNA encoding the protein of the present invention, which is used for quantification of the mRNA encoding the protein, refers to a DNA sequence (antisense) complementary to the genetic DNA (sense) encoding the protein. The aforementioned partial DNA of the genetic DNA encoding the protein of the present invention may consist of a sequence having about 10 to 2200, preferably about 10 to 300, more preferably about 10 to 30 successive nucleotides of the genetic DNA encoding the protein. The aforementioned partial DNA of the cDNA of the genetic DNA encoding the protein refers to a DNA sequence complementary to the partial DNA of the genetic DNA encoding the protein, and may consist of a sequence complementary to a sequence having about 10 to 2200, preferably about 10 to 300, more preferably about 10 to 30 successive nucleotides of the genetic DNA encoding the protein.

The primer, which may be used for the PCR, includes a DNA having the nucleic acid sequence shown by SEQ ID NOs: 5, 6, 7, 8, 9, 10, 11 or 12.

More specifically, the quantification of the mRNA encoding the protein of the present invention can be carried out as follows.
(i) A normal non-human mammal or a non-human mammal disease model (e.g. mouse, rat, rabbit, sheep, swine, bovine, cat, dog, monkey, etc., more specifically, obese mouse, arteriosclerotic mouse, arteriosclerotic rabbit, cancer-bearing mouse) is subjected to a drug administration (e.g. anti-hypertensive, anti-cancer, anti-obesity, anti-hyperlipidemic agent) or a physical stress (e.g. water, electroshock, light and dark, low temp.) loading. After a given period of time, blood, a specified organ (e.g. brain, liver, kidney), or a tissue or a cell isolated from an organ is collected from the animal.
   The mRNA of the protein of the present invention, contained in the thus obtained cells, can be quantified by the TaqMan PCR method or the like, or can be analyzed by the Northern blotting method, after extracting mRNAs from the cells according to a known method.
(ii) A transformant expressing the protein of the present invention can be prepared according to the aforementioned method, and then the mRNA of the protein of the present invention, contained in the transformant, can be quantified in the same way.
   A test compound that reduces the amount of mRNA of the protein of the present invention can be selected as a compound capable of inhibiting the expression of the gene of the protein. A test compound that increases the amount of mRNA of the protein of the present invention can be selected as a compound capable of enhancing the expression of the gene of the protein.

Further, the present invention provides:
(3) a method of screening for a compound or a salt thereof having the activity of enhancing or inhibiting the peptidase activity of the protein of the present invention, which comprises:
   culturing cells (e.g. prostate cancer cells) transformed with a DNA, in which a known promoter or enhancer region derived from the genome DNA of the protein is ligated upstream of an appropriate reporter gene, in the presence of a test compound; and
   measuring the expression of the reporter gene in place of the protein.

The reporter gene may be, for example, a staining marker gene such as lacZ (β-galactosidase gene).

An amount of the reporter gene product is measured according to a known method. A test compound that reduces the amount of the reporter gene product can be selected as a compound capable of inhibiting the expression of the gene of the protein. A test compound that increases the amount of the reporter gene product can be selected as a compound capable of enhancing the expression of the gene of the protein.

These cells may be cultured in the same way as the aforementioned animal cell culture method.

Furthermore, the present invention provides:
(4) a method of screening for a compound or a salt thereof having the activity of enhancing or inhibiting the peptidase activity of the protein of the present invention, which comprises:
   measuring the glutamic acid production by a thin layer chromatography, a column chromatography or the like (i) when culturing the cells capable of expressing the gene of the protein in the presence of an RI-labeled precursor of glutamic acid (specifically, NAAG containing ³H- or ¹⁴C-glutamic acid), and (ii) when culturing the cells capable of expressing the gene of the protein in the presence of the RI-labeled precursor and a test compound; and
   comparing these productions.

These cells may be cultured in the same way as the aforementioned animal cell culture method.

A test compound that reduces the amount of the degradation product such as glutamic acid can be selected as a compound capable of inhibiting the expression of the gene of the protein. A test compound that increases the amount of the degradation product such as glutamic acid can be selected as a compound capable of enhancing the expression of the gene of the protein.

The peptidase activity of the protein of the present invention can be determined according to a well-known method, for example, the method described in M.B. Robinson *et al*., J. Biol. Chem. 262:14498-14506, 1987, or a variant thereof.

A test compound that reduces the peptidase activity of the above case (ii) by about 20 % or more, preferably about 30 % or more, and more preferably about 50 % or more as compared with the above case (i) can be selected as a compound capable of inhibiting the peptidase activity of the protein. A test compound that increases the peptidase activity of the above case (ii) by about 20 % or more, preferably about 30 % or more, and more preferably about 50 % or more as compared with the above case (i) can be selected as a compound capable of enhancing the peptidase activity of the protein.

Furthermore, the present invention provides:
(5) a method of screening for a compound or a salt thereof having the activity of enhancing or inhibiting the peptidase activity of the protein of the present invention, which comprises:
   culturing the cells capable of expressing the gene of the protein in the presence of a test compound; and
   measuring the expression amount of the protein using the antibody to the protein;
and specifically,
(6) a method of screening for a compound or a salt thereof having the activity of enhancing or inhibiting the peptidase activity of the protein of the present invention, which comprises:
   measuring the expression amount of the protein using the antibody to the protein (i) when culturing the cells capable of expressing the gene of the protein and (ii) when culturing the cells capable of expressing the gene of the protein in the presence of a test compound; and
   comparing these expression amounts.

The antibody to the protein of the present invention can be produced by the aforementioned method. The cells can be cultured in the same way as the aforementioned animal cell culture method. And, the expression amount of the protein of the present invention can be determined according to the method of quantifying the protein, as described in the following section [3].

Thus, more specifically, the present invention provides:
(7) a method of screening for a compound or a salt thereof having the activity of enhancing or inhibiting the peptidase activity of the protein of the present invention, which comprises:
   (i) culturing the cells capable of expressing the gene of the protein, and then reacting the antibody to the protein competitively with the culture sample (test sample) and the labeled protein, and
   (ii) culturing the cells capable of expressing the gene of the protein in the presence of a test compound, and then reacting the antibody to the protein competitively with the culture sample (test sample) and the labeled protein; and
      comparing the proportion of the labeled protein bound to the antibody in the cases (i) and (ii);
(8) a method of screening for a compound or a salt thereof having the activity of enhancing or inhibiting the peptidase activity of the protein of the present invention, which comprises:
   (i) culturing the cells capable of expressing the gene of the protein, and then reacting the culture sample (test sample) simultaneously or sequentially with an antibody to the protein immobilized on a carrier, and another labeled antibody to the protein, and
   (ii) culturing the cells capable of expressing the gene of the protein in the presence of a test compound, and then reacting the culture sample (test sample) simultaneously or sequentially with an antibody to the protein immobilized on a carrier, and another labeled antibody to the protein; and
      measuring the activity of the label on the carrier for immobilization in the cases (i) and (ii).

In (8) described above, it is preferred that one antibody recognizes the N-terminal region of the protein of the present invention, and another antibody reacts with the C-terminal region of the protein.

In the screening methods described above, examples of the test compound include peptides, proteins, non-peptide compounds, synthetic compounds, fermentation products, cell extracts, vegetable extracts, animal tissue extracts, and the like, and these compounds may be novel or publicly known.

The screening kit of the present invention comprises the cells capable of expressing the gene of the protein of the present invention, the labeled protein of the present invention, the antibody to the protein of the present invention, and the like.

A compound or a salt thereof to be obtained using the screening method or the screening kit of the present invention is selected from the aforementioned compounds, for example, peptides, proteins, non-peptide compounds, synthetic compounds, fermentation products, cell extracts, vegetable extracts, animal tissue extracts, and blood plasma, and has the activity of enhancing or inhibiting the functions (e.g. peptidase activity) of the protein of the present invention.

The salt of the compound may be any salt form as described above in connection with the salt of the protein of the present invention.

The compound having the activity of inhibiting the functions (e.g. peptidase activity) of the protein of the present invention can be used a therapeutic and/or prophylactic agent for a disease such as cancers (e.g. cancers of stomach, large intestine, rectum, colon, lung, breast, uterine cervix, prostate, ovary, liver, pancreas; chronic lymphatic leukemia, chronic myelocytic leukemia, malignant melanoma, multiple myeloma), neurological diseases (e.g. neurodegenerative diseases such as Alzheimer's disease, schizophrenia, Parkinson's disease, peripheral nerve disease, Huntington's disease, acute brain damage, multiple sclerosis, ALS (amyotrophic lateral sclerosis), peripheral nerve damage, brain ischaemia), and the like.

The compound having the activity of enhancing the functions of the protein of the present invention can be used a therapeutic and/or prophylactic agent for various neurological diseases such as dementia and defect of memory.

When the compound or the salt thereof obtained using the screening method or the screening kit of the present invention is used as the therapeutic and/or prophylactic agents as described above, the administration thereof can follow a conventional manner. For example, the compound can be administered orally or parenterally in a form of tablet, capsule, elixir, microcapsule, aseptic solution, suspension, or the like as described for the pharmaceutical preparation containing the protein of the present invention.

The pharmaceutical preparation thus obtained is safe and low toxic, and can be administered to, for example, a warm-blooded animal (e.g. human, mouse, rat, rabbit, sheep, swine, bovine, horse, bird, cat, dog, monkey, chimpanzee).

The dose of the compound or its salt varies depending on its effect, a target disease, a subject to be administered, a route for administration, etc. For example, when administering orally the compound inhibiting the function of the protein of the present invention to an adult (60 kg body weight) for the treatment of a cancer, the daily dose is normally about 0.1 to 100 mg, preferably about 1.0 to 50 mg, and more preferably about 1.0 to 20 mg of the compound. In parenteral administration, the single dose of the compound also varies depending on a subject to be administered, a target disease, etc. For example, when administering the compound inhibiting the function of the protein of the present invention to an adult (60 kg body weight) for the treatment of a cancer in an injectable form, it is advantageous to inject intravenously the compound at a daily dose of about 0.01 to 30 mg, preferably about 0.1 to 20 mg, and more preferably about 0.1 to 10 mg. For other animal species, the corresponding dose as converted per 60 kg body weight can be administered.

### [3] Quantification of the protein of the present invention, a partial peptide, or a salt thereof

The antibody to the protein of the present invention or the like (hereinafter sometimes referred to as the antibody of the present invention) is capable of specifically recognizing the protein of the present invention. Therefore, the antibody can be used to quantify the protein of the present invention in a test liquid sample, especially for quantification by the sandwich immunoassay.

Thus, the present invention provides the following quantification methods:
(i) a method of quantifying the protein of the present invention in a test liquid sample, which comprises competitively reacting the antibody of the present invention with the test liquid sample and the labeled protein, and measuring the ratio of the labeled protein bound to the antibody; and
(ii) a method of quantifying the protein of the present invention in a test liquid sample, which comprises reacting the test liquid sample with the antibody of the present invention immobilized on a carrier and the labeled another antibody of the present invention simultaneously or sequentially, and measuring the activity of the label on the immobilizing carrier.

In the quantification method (ii) described above, it is preferred that one antibody recognizes the N-terminal region of the protein of the present invention, and the other antibody reacts with the C-terminal region of the protein of the present invention.

Using a monoclonal antibody to the protein of the present invention (hereinafter sometimes referred to as the monoclonal antibody of the present invention), the protein of the present invention can be quantified, and also detected by tissue staining. For this purpose, an antibody molecule itself may be used, or F(ab')₂, Fab' or Fab fractions of the antibody molecule may also be used.

Types of quantification methods using the antibody to the protein of the present invention are not particularly limited. Any assay methods can be used if the amount of antibody, antigen, or antibody-antigen complex corresponding to the amount of antigen (e.g., the amount of the protein) in the test liquid sample can be detected by chemical or physical means and the amount of the antigen can be calculated from a standard curve prepared from standard solutions containing known amounts of the antigen. For example, a nephrometry, a competitive method, an immunometric method, and a sandwich method are appropriately used, with the sandwich method described below being particularly preferable in terms of sensitivity and specificity.

As a labeling agent used for measurement with a labeled substance, there are employed, for example, radioisotopes, enzymes, fluorescent substances, luminescent substances. For the radioisotope, for example, [¹²⁵I], [¹³¹I], [³H] and [¹⁴C] are used. As the enzyme described above, stable enzymes with high specific activity are preferred; for example, β-galactosidase, β-glucosidase, alkaline phosphatase, peroxidase, malate dehydrogenase and the like are used. Examples of the fluorescent substance used are fluorescamine and fluorescein isothiocyanate. For the luminescent substance, for example, luminol, luminol derivatives, luciferin, and lucigenin are used. Furthermore, the biotin-avidin system may be used for coupling of antibody or antigen to the labeling agent.

For immobilization of antigen or antibody, physical adsorption may be used. Chemical binding methods conventionally used for insolubilization or immobilization of proteins or enzymes may also be used. For the carrier, for example, insoluble polysaccharides such as agarose, dextran, cellulose; synthetic resin such as polystyrene, polyacrylamide, silicon; or glass are used.

In the sandwich method, a test liquid sample is reacted with the immobilized monoclonal antibody of the present invention (primary reaction), then with another labeled monoclonal antibody of the present invention (secondary reaction), and the activity of the label on the immobilizing carrier is measured, whereby the amount of the protein of the present invention in the test liquid sample can be quantified. The order of the primary and secondary reactions may be reversed, and these reactions may be performed simultaneously or with an interval. The methods of labeling and immobilization can be performed by the methods described above. In the immunoassay by the sandwich method, the antibody used for immobilized or labeled antibodies is not necessarily one species, but a mixture of two or more species of antibody may be used to increase the measurement sensitivity.

In the quantification of the protein of the present invention by the sandwich method, antibodies that bind to different sites of the protein are preferably used as the monoclonal antibodies of the present invention for the primary and secondary reactions. This means, in respect to the antibodies used for the primary and secondary reactions, if the antibody used in the secondary reaction recognizes the C-terminal region of the protein, the antibody used in the primary reaction preferably recognize a region other than the C-terminal region, for example, the N-terminal region.

The monoclonal antibody of the present invention can be used for the assay systems other than the sandwich method, for example, the competitive method, the immunometric method, and the nephrometry.

In the competitive method, antigen in a test liquid sample and the labeled antigen are competitively reacted with antibody, and the unreacted labeled antigen (F) and the labeled antigen bound to the antibody (B) are separated (B/F separation). The amount of the label in B or F is measured, and the amount of the antigen in the test liquid sample is quantified. This reaction method includes a liquid phase method using a soluble antibody as the antibody, polyethylene glycol for B/F separation and a secondary antibody to the soluble antibody; and a solid phase method either using an immobilized antibody as the primary antibody, or using a soluble antibody as the primary antibody and an immobilized antibody as the secondary antibody.

In the immunometric method, after antigen in a test liquid sample and immobilized antigen are competitively reacted with a given amount of labeled antibody, the solid phase is separated from the liquid phase. Alternatively, after antigen in a test liquid sample and an excess amount of labeled antibody are reacted, and an immobilized antigen is added to bind the unreacted labeled antibody with the solid phase, the solid phase is separated from the liquid phase. Then, the amount of the label in either phase is measured to quantify the antigen in the test liquid sample.

In the nephrometry, an insoluble precipitate produced after the antigen-antibody reaction in gel or solution is quantified. When the amount of antigen in the test liquid sample is small and only a small amount of precipitate is obtained, laser nephrometry using scattering of laser is advantageously employed.

For applying these immunological methods to the quantification method of the present invention, any special conditions or procedures are not required. Systems for quantifying the protein of the present invention are constructed by combining the usual technical consideration in the art to the conventional conditions and procedures. For the details of these general technical means, reference can be made to any reviews and texts.

See, for example, Hiroshi Irie, ed. "Radioimmunoassay" (Kodansha, published in 1974), Hiroshi Irie, ed. "Sequel to the Radioimmunoassay" (Kodansha, published in 1979), Eiji Ishikawa, et al. ed. "Enzyme immonoassay" (Igakushoin, published in 1978), Eiji Ishikawa, et al. ed. "Immunoenzyme assay" (2nd ed.) (Igakushoin, published in 1982), Eiji Ishikawa, et al. ed. "Immunoenzyme assay" (3rd ed.) (Igakushoin, published in 1987), Methods in ENZYMOLOGY, Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies))(all published by Academic Press Publishing).

As described above, the protein of the present invention can be quantified with high sensitivity using the antibody of the present invention.

Furthermore, when an increased level of the protein of the present invention is detected in a patient by quantifying the protein level using the antibody of the present invention, the patient can be diagnosed as highly likely to suffer from, at that time or in the future, a disease such as a cancer (e.g. cancers of stomach, large intestine, rectum, colon, lung, breast, uterine cervix, prostate, ovary, liver, pancreas; chronic lymphatic leukemia, chronic myelocytic leukemia, malignant melanoma, multiple myeloma), a neurological disease (e.g. a neurodegenerative disease such as Alzheimer's disease, schizophrenia, Parkinson's disease, peripheral nerve disease, Huntington's disease, acute brain damage, multiple sclerosis, ALS (amyotrophic lateral sclerosis), peripheral nerve damage, brain ischaemia), etc.

The antibody of the present invention can be employed for detecting the protein of the present invention contained in a test sample such as a body fluid, a tissue. The antibody can also be used for preparing an antibody column suitable for the purification of the protein, for detecting the protein in fractions upon the purification, and for analyzing the behavior of the protein inside a test cell.

A diagnostic agent comprising the antibody of the present invention or a partial peptide thereof having the antigen-binding site is useful for making the diagnosis, prognosis or clinical monitoring of cancers or neurological diseases as described above. In addition, a diagnostic agent kit comprising the antibody of the present invention or the partial peptide thereof having the antigen-binding site may be based on any one of immunohistochemistry, immunocytochemistry, and immunoserology. The agent may be in a liquid form or a powder form, and may also be in a form labeled with an enzyme or a radioisotope. The antibody of the present invention can be used as an in vivo diagnostic agent. In this case, the purified monoclonal antibody is preferred, and the partial peptide thereof having the antigen-binding site, such as Fv, F(ab')₂, Fab fragment (Harlow and Lane, 1988, Antibody, Cold Spring Harbor), the single chain antibody (US patent 4,946,778), or CDR may also be used. A labeling compound is covalently attached to the purified monoclonal antibody directly or through a linker. Various types of labeling compounds, for example, a radioisotope, a fluorescent substance may be used.

### [4] A genetic diagnosis agent

By using the DNA of the present invention as a probe, an aberration (gene aberration) of DNA or mRNA encoding the protein of the present invention or the partial peptide thereof can be detected in a warm-blooded animal (e.g. human, rat, mouse, guinea pig, rabbit, sheep, swine, bovine, horse, cat, dog, monkey, chimpanzee). Therefore, such a probe is useful as a genetic diagnosis agent for detecting damage, mutation, decreased expression, or increased expression or overexpression of said DNA or mRNA.

The genetic diagnosis described above using the DNA of the present invention can be performed by, for example, the well-known Northern hybridization assay or PCR-SSCP assay (Genomics, 5, 874-879 (1989); Proceedings of the National Academy of Sciences of the United States of America, 86, 2766-2770 (1989)).

When the increased expression of the DNA is detected in a pateint by the Northern hybridization method, or the mutation of the DNA is detected by the PCR-SSCP method, the patient can be diagnosed as highly likely to suffer from a disease such as a cancer (e.g. cancers of stomach, large intestine, rectum, colon, lung, breast, uterine cervix, prostate, ovary, liver, pancreas; chronic lymphatic leukemia, chronic myelocytic leukemia, malignant melanoma, multiple myeloma), a neurological disease (e.g. a neurodegenerative disease such as Alzheimer's disease, schizophrenia, Parkinson's disease, peripheral nerve disease, Huntington's disease, acute brain damage, multiple sclerosis, ALS (amyotrophic lateral sclerosis), peripheral nerve damage, brain ischaemia), etc.

### [5] Antisense polynucleotide

An antisense polynucleotide (e.g. antisense DNA), which can be bound complementally to the DNA or mRNA encoding the protein of the present invention to suppress the expression of the DNA, the mRNA, or the protein of the present invention, can suppress in vivo the function of the protein of the present invention as described above or the function of the DNA encoding the protein. Accordingly, the antisense polynucleotide can be used as a prophylactic and/or therapeutic agent for a disease such as a cancer or a neurological disease (e.g. a neurodegenerative disease such as Alzheimer's disease, schizophrenia).

When the antisense polynucleotide is used as said prophylactic and/or therapeutic agent, this agent can be used in the same way as the aforementioned prophylactic and/or therapeutic agent for various diseases comprising the protein or the polynucleotide of the present invention.

Further, the antisense polynucleotide can also be used as a diagnostic oligonucleotide probe to investigate the existence and expression of the polynucleotide of the present invention in tissues or cells.

### [6] A pharmaceutical composition comprising the antibody of the present invention

The antibody of the present invention having the effect to neutralize the activities of the protein of the present invention (the neutralizing antibody) can be used as a prophylactic and/or therapeutic agent for a disease such as a cancer or a neurological disease (e.g. a neurodegenerative disease such as Alzheimer's disease, schizophrenia).

The humanized antibody to the protein of the present invention can be used as a prophylactic and/or therapeutic agent for a disease such as a cancer or a neurodegenerative disease (e.g. Alzheimer's disease, schizophrenia).

The humanized antibody can be produced according to the methods described in Nat Biotechnol. 14, 845-851 (1996); Nat Genet. 15, 146-156 (1997); PNAS 97(2), 722-727 (2000), etc.

In the following of this section [6] "A pharmaceutical composition comprising the antibody of the present invention", the neutralizing antibody and the humanized antibody are generically referred to as the antibody of the present invention.

The therapeutic and/or prophylactic agent for the aforesaid diseases comprising the antibody of the present invention may be administered orally or parenterally to a warm-blooded animal such as a mammal (e.g. human, rat, rabbit, sheep, swine, bovine, cat, dog, monkey) in the original liquid form or in an appropriate pharmaceutical composition form. The dose varies depending on subject to be administered, target disease, conditions, route for administration, etc. For example, when used for the treatment and/or prevention of an adult schizophrenia patient, it is advantageous to inject intravenously the antibody of the present invention in a single dose of normally about 0.01 to 20 mg/kg body weight, preferably about 0.1 to 10 mg/kg body weigh, and more preferably about 0.1 to 5 mg/ kg body weigh at a rate of about 1 to 5 times a day, preferably about 1 to 3 times a day. In another parenteral administration and an oral administration, a dose similar to those given above can be administered. When conditions are serious, the dose may be increased depending on the conditions.

### [7] An anti-tumor agent

It is predicted that the protein of the present invention is increasingly expressed in various cancers, and thus the antibody to the protein or the partial peptide thereof, or an immunotoxin, i.e. a complex of the antibody to the protein or the partial peptide thereof and a toxin is useful as an anti-tumor agent, or a therapeutic and/or prophylactic agent for cancers.

When using the antibody alone by itself, it can attack cancer cells through its complement-dependent or antibody-dependent cytotoxic activity. Alternatively, the antibody may be used along with other chemotherapeutic agents to exhibit the anti-tumor activity in a synergistic manner (Baslya and Mendelsohn, Brest Cancer Res. and Treatment 29, 127-138 (1994)).

In another embodiment, the antibody may be used as a bispecific antibody. The bispecific antibody comprises both the antigen-binding site specific to the protein of the present invention or the partial peptide thereof and a binding site specific to cells having the anti-tumor activity, and it can be produced by a chemical process or a genetic engineering process. Preferred examples of the cells having the anti-tumor activity include cytotoxic T cells, natural killer cells, and macrophages, but any cells having the anti-tumor activity may be used.

On the other hand, the immunotoxin can be produced by covalently coupling of the antibody directly or through a linker to a substance capable of killing cancer cells or inhibiting the proliferation of cancer cells, for example, a radioisotope, an anti-cancer chemotherapeutic agent, or a toxin. Specifically, a number of known methods (US patents 4,671,958; 4,741,900; and 4,867,973) may be employed. For coupling of a chemotherapeutic agent, the method by Chari et al. (US patent 5,208,020) is preferably used. In detail, the antibody to the protein of the present invention or the partial peptide thereof is treated with N-succinimidyl-3-(2-pyridyldithio)propionate according to the method by Carlsson et al. (Biochem. J. 173, 723-737 (1978)) so as to introduce 2-pyridyl disulfide group into the antibody. Then, the modified antibody and a maytansine derivative or May-SH (Cancer Res. 52:127-131, 1992) are mixed, and thus obtained conjugate is purified by gel filtration to remove unreacted May-SH and finally the immunotoxin fraction is obtained. Alternatively, to produce the fusion protein with a toxic protein or peptide, the recombinant DNA technique is preferably used.

The anti-tumor agent comprising the antibody of the present invention may be administered orally or parenterally in the original liquid form or in an appropriate pharmaceutical composition form. The dose varies depending on a subject to be administered, type of cancer, conditions, and route for administration. For example, when used for an adult patient, it is advantageous to inject intravenously a single dose of normally about 0.01 to 20 mg/kg body weight, preferably about 0.1 to 10 mg/kg body weigh, and more preferably about 0.1 to 5 mg/ kg body weigh at a rate of about 1 to 5 times a day, preferably about 1 to 3 times a day. In another parenteral administration and an oral administration, a dose similar to those given above can be administered. When conditions are serious, the dose may be increased depending on the conditions.

### [8] DNA-transfected Animals

The present invention provides a non-human mammal having the foreign DNA encoding the protein of the present invention (abbreviated hereinafter as "the foreign DNA of the present invention") or a mutated DNA thereof (sometimes abbreviated hereinafter as "the foreign mutated DNA of the present invention").

Thus, the invention provides:
(1) A non-human mammal having the foreign DNA of the present invention or the mutated DNA thereof;
(2) The animal described in (1) above, wherein the non-human mammal is a rodent;
(3) The animal described in (2) above, wherein the rodent is a mouse or rat; and
(4) A recombinant vector comprising the foreign DNA of the present invention or the mutated DNA thereof, and having the ability of expressing the DNA in a mammal.

The non-human mammal having the foreign DNA of the present invention or the mutated DNA thereof (hereinafter abbreviated as "the DNA-transfected animal of the present invention") can be prepared by transfecting the desired foreign DNA of the present invention by a method such as the calcium phosphate method, electrical pulse method, lipofection method, agglutination method, microinjection method, particle gun method or DEAE-dextran method into germ cells and the like including unfertilized eggs, fertilized eggs, sperm and primordial cells thereof, preferably during the embryonic stage of non-human mammalian development (and more preferably during the single-cell or fertilized egg cell stage, generally before the eight-cell stage). Such DNA-transfection methods can also be used to transfect the desired foreign DNA of the present invention into somatic cells, living organs or tissue cells for cell culture or tissue culture. The DNA-transfected animal of the present invention can also be produced by fusing these cells with the aforementioned germ cells according to a well-known cell fusion method.

Non-human mammals that can be used include cows, pigs, sheep, goats, rabbits, dogs, cats, guinea pigs, hamsters mice and rats. Among them, from the standpoint of preparing a pathological animal model, a rodent is preferred which has relatively short ontogeny and life cycles and which are easy to breed, especially a mouse (e.g. pure strains such as C57BL/6 and DBA2; and hybrid strains such as B6C3F₁, BDF₁, B6D2F₁, BALB/c and ICR strains) and a rat (such as Wistar and SD strain).

In the context of the recombinant vector which can express the DNA in a mammal, the term "mammal" includes a human as well as a non-human mammal.

The foreign DNA of the present invention refers to the DNA of the present invention that has been previously isolated and extracted from mammals, but not the DNA of the present invention which the non-human mammals have intrinsically.

The mutated DNA of the present invention includes a DNA having a mutation (such as various mutations) in the original nucleotide sequence of the DNA of the present invention, specifically, a DNA having addition, deletion, or substitution of a nucleic acid, and also an abnormal DNA.

The abnormal DNA refers to a DNA which expresses the abnormal protein of the present invention, and includes a DNA which expresses a protein which can enhance the function of the normal protein of the present invention.

The foreign DNA of the present invention may be derived from a mammal of either the same species or different species from the target animal. When transfecting the DNA of the present invention into the target animal, it is generally advantageous to use a DNA construct having the DNA ligated downstream of a promoter which can function in the animal cell. For example, when transfecting the human DNA of the present invention, a DNA-transfected mammal can be prepared, which highly expresses the DNA of the present invention, by microinjecting into the fertilized eggs of the target mammal, such as fertilized mouse eggs, a DNA construct (such as a vector) having the human DNA of the present invention ligated downstream of various promoters which can express a DNA derived from various mammals (such as rabbits, dogs, cats, guinea pigs, hamsters, rats or mice) having the DNA of the present invention, which is highly homologous to the human DNA.

Plasmids derived from *E. coli, B. subtilis* or yeast, bacteriophages such as λ-phage, retroviruses such as Moloney leukemia virus and animal viruses such as vaccinia virus and baculovirus may be used as the expression vector of the protein of the present invention. Among them, plasmids derived from *E. coli, B. subtilis* or yeast are preferred.

Promoters that can be used to regulate the DNA expression include (i) promoters derived from viruses (such as simian virus, cytomegalovirus, Moloney leukemia virus, JC virus, mammary tumor virus or polio virus), and (ii) promoters derived from various mammals (humans, rabbits, dogs, cats, guinea pigs, hamsters, rats, mice, etc.), such as promoters of albumin, insulin II, uroplakin II, elastase, erythropoietin, endothelin, muscle creatine kinase, glial fibrillary acidic protein, glutathione S-transferase, platelet derived growth factor β, keratin K1, K10 and K14, collagen Type I and Type II, cyclic AMP-dependent protein kinase βI subunit, dystrophin, tartaric acid-resistant alkali phosphatase, cardiac sodium diuretic factor, endothelial receptor tyrosine kinase (normally abbreviated as Tie2), sodium-potassium ATPase (Na,K-ATPase), neurofilament light chain, metallothionein I and IIA, tissue inhibitor of metalloproteinase-1, MHC class I antigen (H-2L), H-ras, renin, dopamine β-hydroxylase, thyroid peroxidase (TPO), polypeptide chain elongation factor 1α (EF-1α), β-actin, α- and β-myosin heavy chains, myosin light chains 1 and 2, myelin basic protein, thyroglobulin, Thy-1, immunoglobulin, H chain variable region (VNP), serum amyloid P component, myoglobin, troponin C, smooth muscle α-actin, preproenkephalin A, vasopressin. Particularly suitable are cytomegalovirus promoter, human polypeptide chain elongation factor 1α (EF- 1α) promoter and human and chicken β-actin promoters, which allow strong expression throughout the body.

The said vectors should preferably have the sequence (generally called the terminator) which terminates transcription of the target mRNA in DNA-transfected mammals. Terminator DNA sequences derived from viruses and mammals can be used, and the simian virus SV40 terminator is preferably used.

In order to achieve greater expression of the desired foreign DNA, it is also possible depending on the purpose to attach various DNA splicing signals, enhancer regions or parts of eukaryote-derived DNA introns at 5'-upstream of the promoter region, between the promoter region and the translation region, or at 3'-downstream of the translation region.

The translation region of the normal protein of the present invention may be obtained as a whole or part of genomic DNA from DNAs derived from heart, kidney, pancreas, or prostate of various mammals (e.g. humans, rabbits, dogs, cats, guinea pigs, hamsters, rats, mice) or from various commercial genomic DNA libraries, or may be obtained from complement DNAs prepared by a well-known method from RNAs derived from heart, kidney, pancreas, or prostate. To prepare the abnormal foreign DNA, the translation region of normal protein obtained from the aforementioned cells or tissues can be mutated by point mutagenesis.

A DNA construct enabling the expression of the translation region in the DNA-transfected animal can be produced by a conventional genetic engineering method of inserting the translation region after the aforementioned promoter, or if desired, before the transcription termination site.

Transfection of the foreign DNA of the present invention at the fertilized egg cell stage ensures that the DNA of the present invention will be present in all germ and somatic cells of the target mammal. The presence of the foreign DNA of the present invention in the animal's germ cells after the DNA transfection means that all the animal's progenies will retain the foreign DNA of the present invention in all their germ and somatic cells. The progenies of this animal that inherit the foreign DNA of the present invention have the DNA in all their germ and somatic cells.

The non-human mammal into which the normal foreign DNA of the present invention has been transfected can be bred after confirmation of stable retention of the foreign DNA, and can be successively reared in a normal environment as an animal retaining the DNA.

Transfection of the foreign DNA of the present invention at the fertilized egg cell stage ensures that the DNA of the present invention will be present in excess in all germ and somatic cells of the target mammal. The excessive presence of the foreign DNA of the present invention in the animal's germ cells after the DNA transfection means that all the animal's progenies will retain an excess of the foreign DNA of the present invention in all their germ and somatic cells. The progenies of this animal that inherit the foreign DNA of the present invention have an excess of the foreign DNA of the present invention in all their germ and somatic cells.

It is possible to obtain homozygotic animals having the transfected DNA in both homologous chromosomes, and to breed the male and female so that all the progenies have the DNA in excess.

The normal DNA of the present invention is highly expressed in the non-human mammal having the normal DNA of the present invention, leading to the promotion of the function of the intrinsic normal DNA and ultimately to the hyperfunction of the protein of the present invention. Such an animal is useful as a pathological animal model. For example, the normal DNA-transfected animal can be used to elucidate the pathology of hyperfunction of the protein of the present invention and other diseases related to the protein of the present invention, and to investigate therapies for these conditions.

Furthermore, since the mammal into which the normal foreign DNA of the present invention is transfected has symptoms due to increased free protein of the present invention, it can also be used in screening tests for pharmaceuticals for treatment of conditions related to the protein of the present invention.

The non-human mammal having the abnormal foreign DNA of the present invention can be bred after confirmation of stable retention of the foreign DNA, and can be successively reared in a normal environment as an animal retaining the DNA. Furthermore, the desired foreign DNA can be incorporated into one of the aforementioned plasmids and used as a material. A DNA construct with a promoter can be produced according to ordinary DNA engineering techniques. Transfection of the abnormal DNA of the present invention at the fertilized egg stage ensures that the abnormal DNA of the present invention is present in all the germ and somatic cells of the target mammal. The presence of the abnormal DNA of the present invention in the animal's germ cells after the DNA transfection means that all the animal's progenies will retain the abnormal DNA of the present invention in all their germ and somatic cells. The progenies of this animal that inherit the foreign DNA will have the abnormal DNA of the present invention in all their germ and somatic cells. It is possible to obtain homozygote animals having the transfected DNA in both homologous chromosomes, and to breed the male and female so that all the progenies have this DNA.

The abnormal DNA of the present invention is highly expressed in the non-human mammal having the abnormal DNA of the present invention, leading to the inhibition of the function of the intrinsic normal DNA, and ultimately to the dysfunction of the protein of the present invention. Such an animal is useful as a pathological animal model. For example, the abnormal DNA-transfected animal can be used to elucidate the pathology of dysfunction of the protein of the present invention, and to investigate therapies for this condition.

In a specific possible application, the animal that highly expresses the abnormal DNA of the present invention could be a model for elucidating the inhibitory mechanism of normal protein function (dominant negative effect) mediated by the abnormal protein in the dysfunction of the protein of the present invention.

Moreover, since the mammal into which the abnormal foreign DNA of the present invention is transfected has symptoms due to increased free protein of the present invention, it can also be used in screening tests for pharmaceuticals for treatment of dysfunction of the protein of the present invention.

Other possible applications of the said two types of DNA-transfected animals of the present invention include:
(1) use as cell sources for tissue culture;
(2) direct analysis of DNA or RNA in the tissue of DNA-transfected mammals of the present invention or analysis of proteins expressed in tissues to elucidate the involvement of proteins that are specifically expressed or activated by the protein of the present invention;
(3) researching the function of cells derived from a tissue which is generally difficult to culture, by using cells derived from a tissue having the DNA of the present invention, wherein such cells can be cultured by standard tissue culture techniques;
(4) screening for pharmaceuticals that enhance the cellular functions using the cells described in (3) above; and
(5) isolation and purification of the mutated protein of the present invention, and production of antibodies thereto.

The DNA-transfected animals of the present invention could also be used to investigate the clinical symptoms of diseases related to the protein of the present invention, including dysfunction of the protein of the present invention, to obtain more detailed pathologies of various organs of the disease models related to the protein of the present invention, to develop new therapies, and to contribute to research and therapies for secondary conditions stemming from such diseases.

It is also possible to remove various organs from the DNA-transfected animals of the present invention, mince them, treat them with a protease such as trypsin to obtain free DNA-transfected cells, and culture the cells to prepare a cell line from the cultured cells. Since it is possible to specify the cells producing the protein of the present invention, and investigate the cells for apoptosis, differentiation and proliferation, and signal transduction, the cells can be effective research materials for understanding the protein of the present invention and action thereof.

Moreover, the DNA-transfected animals of the present invention may also be used to provide a rapid method of screening for a pharmaceutical for the treatment of diseases related to the protein of the present invention, including dysfunction of the protein of the present invention in the drug development using the assay methods and the quantifying method as described above. The DNA-transfected animals of the present invention or the vectors expressing the foreign DNA of the present invention may also be used to investigate and develop DNA therapies for diseases related to the protein of the present invention.

### [9] Knockout Animals

The present invention provides a non-human mammal embryonic stem cell in which the DNA of the present invention is inactivated; and a non-human mammal which fail to express the DNA of the present invention.

Thus, the present invention provides:
(1) a non-human mammal embryonic stem cell in which the DNA of the present invention is inactivated;
(2) the embryonic stem cell according to (1), wherein the DNA is inactivated by inserting a reporter gene (e.g., β-galactosidase gene derived from *Escherichia coli*);
(3) the embryonic stem cell according to (1), which is resistant to neomycin;
(4) the embryonic stem cell according to (1), wherein the non-human mammal is a rodent;
(5) the embryonic stem cell according to (4), wherein the rodent is a mouse;
(6) a non-human mammal deficient in expressing the DNA of the present invention, wherein the DNA of the present invention is inactivated;
(7) the non-human mammal according to (6), wherein the DNA is inactivated by inserting a reporter gene (e.g., β-galactosidase derived from *Escherichia coli*) therein and the reporter gene is capable of being expressed under control of a promoter for the DNA of the present invention;
(8) the non-human mammal according to (6), which is a rodent;
(9) the non-human mammal according to (8), wherein the rodent is a mouse; and,
(10) a method for screening a compound that enhances or inhibits the promoter activity for the DNA of the present invention, which comprises administering a test compound to the mammal of (7) and detecting expression of the reporter gene.

The non-human mammal embryonic stem cell in which the DNA of the present invention is inactivated refers to the embryonic stem cells (abbreviated hereinafter as "ES cells") of a non-human mammal either in which the DNA expression ability is suppressed by the addition of an artificial modification to the DNA of the present invention in the non-human mammal, or in which the activity of the protein of the present invention encoded by said DNA has substantially been eliminated so that the DNA is not substantially capable of expressing the protein of the present invention (sometimes referred to hereinafter as the knockout DNA of the present invention).

The non-human mammals to be used are as described above.

The method of artificially modifying the DNA of the present invention includes one using genetic engineering techniques to delete a whole or a part of the DNA sequence, or to insert or substitute other DNA. The knockout DNA of the present invention is produced by the modification of shifting the codon reading frame or of disrupting the function of the promoter or exon.

Specifically, the non-human mammal embryonic stem cell in which the DNA of the present invention is inactivated (abbreviated hereinafter as ES cells of the present invention comprising inactivated DNA or knockout ES cells of the present invention) can be produced as follows. For example, the DNA of the present invention in the target non-human mammal is isolated, a drug-resistant gene of which typical examples are neomycin-resistant, hygromycin-resistant or other drug-resistant genes, or a reporter gene or the like of which typical examples are 1acZ (β-galactosidase gene) or cat (chloramphenicol acetyltransferase gene) is inserted into the exon to disrupt the function of the exon, or else a DNA sequence (such as polyA addition signal) which terminates gene transcription is inserted into the intron between the exons to completely prevent mRNA synthesis. A DNA chain having the thus constructed DNA sequence to disrupt the gene (abbreviated hereinafter as "the targeting vector") is introduced into the chromosomes of the animal by homologous recombination. The knockout ES cell of the present invention can be selected by analyzing the thus obtained ES cells either by the southern hybridization using a DNA sequence on or near the DNA of the present invention as a probe, or by the PCR using as primers a DNA sequence on the targeting vector and a DNA sequence of a nearby region of the DNA of the present invention used in producing the targeting vector.

For ES cells originally used to inactivate the DNA of the present invention by homologous recombination, it is possible to use already established cells as described above, or to establish a new one according to the known Evans and Kaufman methods. For example, the mouse 129 ES cell line is currently in general use, but the immunological background is unclear. Accordingly, to establish another pure line for which the immunological and genetic background is clear, it is good to use C57BL/6 mice or else BDF1 mice (F1 of C57BL/6 and DBA/2) in which the low egg recovery of C57BL/6 mice is improved by cross-breeding with DBA/2 mice. Not only do BDF1 mice show high egg recovery and sturdy eggs, but they are based on C57BL/6 mice, and thus the genetic background of ES cells obtained therefrom can be restored by back crossing with C57BL/6 mice when preparing a disease model mouse.

A blastocyst 3.5 days after fertilization is generally used in establishing ES cells, but many early embryos can be obtained efficiently by collecting 8-cell embryos and culturing them to the blastocyst stage.

Either female or male ES cells can be used, but generally male ES cells are more useful for preparing reproductive lineage chimeras. Females and males should be distinguished as quickly as possible to reduce laborious culture work.

One method of distinguishing female and male ES cells is to use PCR to amplify and detect the genes of the sex-determining region on the Y chromosome. Previously, about 10⁶ cells were required for karyotype analysis, but this method uses only about one colony of ES cells (about 50 cells), allowing primary selection of ES cells by determining cell sex at the initial culture stage. Early selection of male cells can greatly cut the work of the initial culture stage.

Secondary selection can be accomplished for example by using the G-banding method to confirm the number of chromosomes. Ideally, 100% of the ES cells should have a normal number of chromosomes. When this is difficult due to the physical manipulation used in establishing the cells, the ES cell gene should be cloned again into a normal cell (for example, those having the normal mouse chromosome number of 2n = 40) after the ES cell gene is knocked out.

In general, the resulting embryonic stem cell line is highly productive, but since it can easily lose the power of ontogenesis, successive cultures must be performed very carefully. For example, culture can be performed in a carbon dioxide incubator (preferably with 5% carbon dioxide, 95% air or 5% oxygen, 5% carbon dioxide, 90% air) at about 37°C in the presence of LIF (1-10000 U/ml) on suitable feeder cells such as STO fibroblasts. During passage, treatment with a trypsin/EDTA solution (normally 0.001-0.5% trypsin/0.1-5 mM EDTA, preferably about 0.1% trypsin/1 mM EDTA) is used to produce a single cell, which is then seeded on a newly prepared feeder cells. Such a passage is normally performed every 1-3 days, and simultaneously, the cells should be monitored. If the cells are found morphologically abnormal, it is desired to discard the cultured cells.

ES cells can be differentiated into a variety of cells types, such as musculus longus capitis, visceral muscle or cardiac muscle cells, by culturing under suitable conditions either in a monolayer culture until they reach high density, or else in a floating culture until they form a cell clump (M.J. Evans and M. H. Kaufman, *Nature*, Vol. 292, 154 (1981); G. R. Martin, *Proc. Natl. Acad. Sci. USA*, Vol. 78, 7634 (1981); T. C. Doetschman et al, *Journal of Embryology and Experimental Morphology*, Vol. 87, 27 (1985). The cells that fail to express the DNA of the present invention, obtained by differentiating the ES cells of the present invention, are useful in investigating *in vitro* cellular functions of the protein of the present invention.

The non-human mammal that fails to express the DNA of the present invention can be distinguished from normal animals by measuring the amount of mRNA by a well-known method to indirectly compare the expression amount.

The non-human mammals to be used are as described above.

In the non-human mammal that fails to express the DNA of the present invention, the DNA of the present invention can be knocked out for example by introducing a targeting vector created as described above into mouse embryonic stem cells or mouse egg cells, resulting in the replacement, by genetic homologous recombination, of the DNA of the present invention on the chromosomes of the mouse's embryonic stem cells or egg cells with the DNA sequence in the targeting vector in which the DNA of the present invention is inactivated.

The cells in which the DNA of the present invention is knocked out can be evaluated either by the southern hybridization method using a DNA sequence on or near the DNA of the present invention as a probe, or by the PCR method using as primers a DNA sequence on the targeting vector and the DNA sequence of a nearby region of the mouse-derived DNA of the present invention used in creating the targeting vector. When using non-human mammal embryonic stem cells, a cell line in which the DNA of the present invention is inactivated can be cloned by homologous recombination, and the cells injected into the embryos or blastocysts of a non-human mammal at a suitable stage such as the 8-cell stage. The resulting chimera embryo is then transplanted to the uterus of the non-human mammal, which has been made falsely pregnant. The resulting animal is a chimera animal comprising both cells with the normal DNA locus of the present invention and the artificially mutated DNA locus of the present invention.

If some of the reproductive cells of this chimera animal have the mutated DNA of the present invention, individuals all of whose tissues are made up of cells having the artificially modified DNA locus of the present invention can be selected by evaluation of coat color, for example, from a population produced by the breeding of this chimera with a normal individual. The individual obtained in this way normally is usually one which heterozygously fails to express the protein of the present invention. Thus, by breeding such heterozygously knocked-out animals, it is possible to obtain from their offspring an individual that homozygously fails to express the protein of the present invention.

When using egg cells, it is possible to obtain a transgenic non-human mammal having the targeting vector inserted into the chromosomes by injecting the DNA solution into an egg cell nucleus with microinjection. From these transgenic non-human mammals, selected is one having the mutation on the DNA locus of the present invention due to homologous recombination.

The animal in which the DNA of the present invention has been knocked out in this way can be successively reared in a normal environment after confirmation that the DNA is knocked out in its offsprings obtained by breeding.

Reproductive lineages can also be obtained and maintained by ordinary methods. Thus, female and male animals having the inactivated DNA can be bred to obtain homozygous animals with the inactivated DNA in both homologous chromosomes. The resulting homozygous animals can be efficiently reproduced by rearing under the condition of one normal individual and multiple homozygote individuals to a mother animal. By breeding female and male heterozygous animals, homozygous and heterozygous animals having the inactivated DNA are successively produced.

The non-human mammal embryonic stem cell in which the DNA of the present invention is inactivated is extremely useful in preparing the non-human mammal which fails to express the DNA of the present invention.

Moreover, because the non-human mammal which fails to express the protein of the present invention lacks various kinds of biological activities which may be induced by the protein of the present invention, it can be a model of various diseases stemming from inactivation of the biological activities of the protein of the present invention, and is therefore useful for examining causes and therapies for such diseases.

### [9a] A method for screening of a compound having a therapeutic/prophylactic effect for diseases caused by deficiency, damages, etc. of the DNA of the present invention

The non-human mammal deficient in expression of the DNA of the present invention can be employed for screening of a compound having therapeutic/prophylactic effects for diseases caused by deficiency, damages, etc. of the DNA of the present invention (e.g. neurological diseases such as dementia and defect of memory).

Thus, the present invention provides a method for screening of a compound having therapeutic/prophylactic effects for diseases caused by deficiency, damages, etc. of the DNA of the present invention, which comprises administering a test compound to the non-human mammal deficient in expression of the DNA of the present invention, and monitoring a change occurred in the animal.

As the non-human mammal deficient in expression of the DNA of the present invention which can be employed for the screening method, the same examples as given hereinabove apply.

Examples of the test compound include peptides, proteins, non-peptide compounds, synthetic compounds, fermentation products, cell extracts, vegetable extracts, animal tissue extracts, blood plasma and the like, and these compounds may be novel or publicly known.

Specifically, after treating the non-human mammal deficient in expression of the DNA of the present invention with a test compound, and making a comparison with an intact control animal, a change in each organ, tissue, disease conditions, etc. of the animal is used as an index to assess the therapeutic/prophylactic effects of the test compound.

The method of treating an test animal with a test compound includes oral administration, intravenous injection, etc., and it is appropriately selected depending upon conditions of the test animal, properties of the test compound, etc. In addition, the dose of the test compound can be appropriately selected depending on the administration route, nature of the test compound and the like.

For example, for the screening of a compound having a therapeutic/prophylactic effect on defect of memory, the non-human mammal deficient in expression of the DNA of the present invention is subjected to a physical stress loading. A test compound is administered before or after the stress loading, and the change in stress-avoidance behavior of the animal is measured with time.

In the screening method *supra*, when a test compound is administered to an test animal and then found to improve the stress-avoidance behavior of the animal by at least about 10 %, preferably at least about 30 %, and more preferably at least about 50 %, the test compound can be selected to be a compound having a therapeutic and prophylactic effect on defect of memory.

The compound obtained using the above screening method is selected from test compounds as described above and exhibits a therapeutic and prophylactic effect on the diseases caused by deficiencies, damages, etc. of the protein of the present invention (e.g. neurological diseases such as dementia and defect of memory). Therefore, the compound can be employed as a safe and low-toxic therapeutic and/or prophylactic agent for the diseases. Furthermore, a derivative from the compound obtained by the screening *supra* can be likewise employed.

The compound obtained by the screening above may be used in a salt form with a physiologically acceptable acid (e.g., inorganic acids or organic acids) or base (e.g., alkali metals), preferably in the form of a physiologically acceptable acid addition salt. Examples of such salts include salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

A pharmaceutical composition comprising the compound obtained by the above screening method or a salt thereof may be manufactured in a manner similar to the method for preparing the composition comprising the protein of the present invention as described above. Since the pharmaceutical composition thus obtained is safe and low toxic, it can be administered to a human and other mammals (e.g. rat, mouse, guinea pig, rabbit, sheep, swine, bovine, horse, cat, dog, monkey).

The dose of the compound or the salt thereof varies depending on a target disease, a subject to be administered, a route for administration, etc. For example, in oral administration of the compound to an adult (60 kg body weight) for the treatment of dementia, the daily dose is normally about 0.1 to 100 mg, preferably about 1.0 to 50 mg, and more preferably about 1.0 to 20 mg of the protein. In parenteral administration, the single dose also varies depending on a subject to be administered, a target disease, etc., and it is advantageous to administer the compound intravenously at a daily dose of about 0.01 to 30 mg, preferably about 0.1 to 20 mg, and more preferably about 0.1 to 10 mg to an adult (60 kg body weight) for the treatment of dementia. For other animal species, the corresponding dose as converted per 60 kg body weight can be administered.

### [9b] A method for screening a compound that can enhance or inhibit the activity of the promoter for the DNA of the present invention

The present invention provides a method for screening a compound that can enhance or inhibit the activity of the promoter for the DNA of the present invention or a salt thereof, which comprises administering a test compound to the non-human mammal deficient in expression of the DNA of the present invention and detecting expression of the reporter gene.

In the screening method *supra*, used is the non-human mammal deficient in expression of the DNA of the present invention in which the DNA of the present invention is inactivated by introducing a reporter gene and the reporter gene is expressed under control of the promoter for the DNA of the present invention.

Examples of the test compound are as described above.

Examples of the reporter gene are as described above. Preferably employed are β-galactosidase gene (lacZ), soluble alkaline phosphatase gene, luciferase gene and the like.

In the non-human mammal deficient in expression of the DNA of the present invention wherein the DNA is substituted with the reporter gene, since the reporter gene is present under control of the promoter for the DNA of the present invention, the activity of the promoter can be detected by monitoring the expression of the substance encoded by the reporter gene.

For example, when a part of the DNA region encoding the protein of the present invention is substituted with β-galactosidase gene (lacZ) derived from *Escherichia coli*, β-galactosidase is expressed in place of the protein of the present invention in a tissue where the protein of the present invention should originally be expressed. Thus, the expression state of the protein of the present invention can be readily observed in an animal body by staining with a reagent, e.g. 5-bromo-4-chloro-3-indolyl-β-galactopyranoside (X-gal) which is substrate for β-galactosidase. Specifically, a mouse deficient in the protein of the present invention, or its tissue section is fixed with glutaraldehyde, washed with phosphate buffered saline (PBS), and then incubated with a staining solution containing X-gal at room temperature or about 37°C for about 30 minutes to an hour. After the β-galactosidase reaction is terminated by washing the tissue preparation with 1 mM EDTA/PBS solution, the color change is observed. Alternatively, the mRNA encoding 1acZ may be detected in a conventional manner.

The compound or a salt thereof obtained using the screening method *supra* is selected from the test compounds described above and can enhance or inhibit the promoter activity for the DNA of the present invention.

The compound obtained by the screening above may be used in a salt form with a physiologically acceptable acid (e.g., inorganic acids or organic acids) or base (e.g., alkali metals), preferably in the form of a physiologically acceptable acid addition salt. Examples of such salts include salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The compound or the salt thereof that can inhibit the promoter activity for the DNA of the present invention can inhibit the expression of the protein of the present invention, and finally inhibit the function of the protein of the present invention. Accordingly, it is useful as a safe and low-toxic therapeutic and/or prophylactic agent for a disease such as a cancer (e.g. cancers of stomach, large intestine, rectum, colon, lung, breast, uterine cervix, prostate, ovary, liver, pancreas; chronic lymphatic leukemia, chronic myelocytic leukemia, malignant melanoma, multiple myeloma), a neurodegenerative disease (e.g. Alzheimer's disease, schizophrenia, Parkinson's disease, peripheral nerve disease, Huntington's disease, acute brain damage, multiple sclerosis, ALS (amyotrophic lateral sclerosis), peripheral nerve damage, brain ischaemia) and the like.

The compound or a salt thereof that can enhance the promoter activity for the DNA of the present invention can enhance the expression of the protein of the present invention, and finally enhance the function of the protein of the present invention. Accordingly, it is useful as a safe and low-toxic therapeutic and/or prophylactic agent for a neurological disease such as dementia and defect of memory.

A pharmaceutical composition comprising the compound or a salt thereof obtained by the screening method *supra* may be manufactured in a manner similar to the method for preparing the composition comprising the protein of the present invention as described above.

Since the pharmaceutical composition thus obtained is safe and low toxic, it can be administered to a human or another mammal (e.g. rat, mouse, guinea pig, rabbit, sheep, swine, bovine, horse, cat, dog, monkey).

The dose of the compound or the salt thereof varies depending on a target disease, a subject to be administered, a route for administration, etc. For example, in oral administration of the compound inhibiting the promoter activity for the DNA of the present invention to an adult (60 kg body weight) for the treatment of a cancer, the daily dose is normally about 0.1 to 100 mg, preferably about 1.0 to 50 mg, more preferably about 1.0 to 20 mg for adult (as 60 kg body weight). In parenteral administration, the single dose of the compound also varies depending on a subject to be administered, target disease, etc. For example, it is advantageous to administer intravenously in an injectable form the compound inhibiting the promoter activity for the DNA of the present invention at a daily dose of about 0.01 to 30 mg, preferably about 0.1 to 20 mg, more preferably about 0.1 to 10 mg to an adult (60 kg body weight) for the treatment of a cancer. For other animal species, the corresponding dose as converted per 60 kg weight can be administered.

As stated above, the non-human mammal deficient in expression of the DNA of the present invention is extremely useful for screening the compound or the salt thereof that can enhance or inhibit the activity of the promoter for the DNA of the present invention, and can greatly contribute to the elucidation of causes for various diseases related to deficiency in expression of the DNA of the present invention, and to the development of a prophylactic/therapeutic agent for the diseases.

Furthermore, in case that a so-called transgenic animal (gene-transferred animal) is prepared by ligating various protein-coding genes downstream to a DNA sequence containing the promoter region for the protein of the present invention and injecting the same into an animal egg, it can be used to study the in vivo functions of such protein which can be expressed in a specific manner. As well, in case that a cell line is established in which an appropriate reporter gene is ligated to the said promoter site, it can be used as a research system for a low-molecular weigh compound capable of inhibiting specifically the in vivo production of the protein of the present invention. It is also possible to search for a novel cis-element and a transcriptional factor capable of binding to the element through analysis of the promoter site.

In the specification and drawings, abbreviations of bases and amino acids are based on the abbreviations of the IUPAC-IUB Commission on Biochemical Nomenclature or the conventional abbreviations used in the art, examples of which are shown below. An amino acid that has an optical isomer takes its L form unless otherwise indicated.

| | |
|---|---|
| DNA | deoxyribonucleic acid |
| cDNA | complementary deoxyribonucleic acid |
| A | adenine |
| T | thymine |
| G | guanine |
| C | cytosine |
| RNA | ribonucleic acid |
| mRNA | messenger ribonucleic acid |
| dATP | deoxyadenosine triphosphate |
| dTTP | deoxythymidine triphosphate |
| dGTP | deoxyguanosine triphosphate |
| dCTP | deoxycytidine triphosphate |
| ATP | adenosine triphosphate |
| EDTA | ethylenediaminetetraacetic acid |
| SDS | sodium dodecyl sulfate |
| Gly | glycine |
| Ala | alanine |
| Val | valine |
| Leu | leucine |
| Ile | isoleucine |
| Ser | serine |
| Thr | threonine |
| Cys | cysteine |
| Met | methionine |
| Glu | glutamic acid |
| Asp | aspartic acid |
| Lys | lysine |
| Arg | arginine |
| His | histidine |
| Phe | phenylalanine |
| Tyr | tyrosine |
| Trp | tryptophan |
| Pro | proline |
| Asn | asparagine |
| Gln | glutamine |
| pGlu | pyroglutamic acid |

The substituents, protective groups and reagents, which are frequently used throughout the specification, are shown by the following abbreviations.

| | |
|---|---|
| Me | methyl |
| Et | ethyl |
| Bu | butyl |
| Ph | phenyl |
| TC | thiazolidine-4(R)-carboxamide |
| Tos | p-toluenesulfonyl |
| CHO | formyl |
| Bzl | benzyl |
| Cl₂-Bzl | 2,6-dichlorobenzyl |
| Bom | benzyloxymethyl |
| Z | benzyloxycarbonyl |
| Cl-Z | 2-chlorobenzyloxycarbonyl |
| Br-Z | 2-bromobenzyloxycarbonyl |
| Boc | t-butoxycarbonyl |
| DNP | dinitrophenol |
| Trt | trityl |
| Bum | t-butoxymethyl |
| Fmoc | N-9-fluorenylmethoxycarbonyl |
| HOBt | 1-hydroxybenztriazole |
| HOOBt | 3,4-dihydro-3-hydroxy-4-oxo-1,2,3-benzotriazine |
| HONB | 1-hydroxy-5-norbornene-2,3-dicarboximide |
| DCC | N,N'-dicyclohexylcarbodiimide |

The SEQ ID NOs (sequence identification numbers) in the Sequence Listing of the present specification indicate the following sequences, respectively.

### [SEQ ID NO: 1]

This shows the amino acid sequence of the human-derived peptidase protein of the present invention.

### [SEQ ID NO: 2]

This shows the nucleic acid sequence of the DNA encoding the human-derived the human-derived peptidase protein of the present invention having the amino acid sequence shown by SEQ ID NO: 1.

### [SEQ ID NO: 3]

This shows the amino acid sequence of the human-derived peptidase protein of the present invention.

### [SEQ ID NO: 4]

This shows the nucleic acid sequence of the DNA encoding the human-derived the human-derived peptidase protein of the present invention having the amino acid sequence shown by SEQ ID NO: 3.

### [SEQ ID NO: 5]

This shows the nucleic acid sequence of the synthetic primer 1 used in Example I for the cloning of the DNA encoding the human-derived the human-derived protein of the present invention.

### [SEQ ID NO: 6]

This shows the nucleic acid sequence of the synthetic primer 2 used in Example 1 for the cloning of the DNA encoding the human-derived the human-derived protein of the present invention.

### [SEQ ID NO: 7]

This shows the nucleic acid sequence of the synthetic primer 3 used in Example 1 for the cloning of the DNA encoding the human-derived the human-derived protein of the present invention.

### [SEQ ID NO: 8]

This shows the nucleic acid sequence of the synthetic primer 4 used in Example 1 for the cloning of the DNA encoding the human-derived the human-derived protein of the present invention.

### [SEQ ID NO: 9]

This shows the nucleic acid sequence of the synthetic primer 5 used in Example 1 for the cloning of the DNA encoding the human-derived the human-derived protein of the present invention.

### [SEQ ID NO: 10]

This shows the nucleic acid sequence of the synthetic primer 6 used in Example 1 for the cloning of the DNA encoding the human-derived the human-derived protein of the present invention.

### [SEQ ID NO: 11]

This shows the nucleic acid sequence of the synthetic primer 7 used in Example 2 for the cloning of the DNA encoding the human-derived the human-derived protein of the present invention.

### [SEQ ID NO: 12]

This shows the nucleic acid sequence of the synthetic primer 8 used in Example 2 for the cloning of the DNA encoding the human-derived the human-derived protein of the present invention.

### [SEQ ID NO: 13]

This shows the amino acid sequence of the human-derived peptidase protein of the present invention.

### [SEQ ID NO: 14]

This shows the nucleic acid sequence of the DNA encoding the human-derived the human-derived peptidase protein of the present invention having the amino acid sequence shown by SEQ ID NO: 13.

### [SEQ ID NO: 15]

This shows the nucleic acid sequence of the synthetic primer 9 used for the PCR in Example 3.

### [SEQ ID NO: 16]

This shows the nucleic acid sequence of the synthetic primer 10 used for the PCR in Examples 3 and 7.

### [SEQ ID NO: 17]

This shows the amino acid sequence of the synthetic peptide 1 used in Example 6.

### [SEQ ID NO: 18]

This shows the amino acid sequence of the synthetic peptide 2 used in Example 6.

### [SEQ ID NO: 19]

This shows the nucleic acid sequence of the synthetic primer 11 used for the PCR in Example 7.

### [SEQ ID NO: 20]

This shows the nucleic acid sequence of the synthetic primer 12 used for the PCR in Example 9.

### [SEQ ID NO: 21]

This shows the nucleic acid sequence of the synthetic primer 13 used for the PCR in Example 9.

### [SEQ ID NO: 22]

This shows the nucleic acid sequence of the synthetic primer 14 used in Example 10 for the comparison of expression amounts of the gene by TaqMan method.

### [SEQ ID NO: 23]

This shows the nucleic acid sequence of the synthetic primer 15 used in Example 10 for the comparison of expression amounts of the gene by TaqMan method.

### [SEQ ID NO: 24]

This shows the nucleic acid sequence of the synthetic TaqMan probe used in Example 10 for the comparison of expression amounts of the gene by TaqMan method.

The transformant *Escherichia coli* DH5α/pTB2186 obtained in Example 2 is on deposit with International Patent Organism Depositary, National Institute of Advanced Industrial Science and Technology (the successor of National Institute of Bioscience and Human Technology (NIBH), Agency of Industrial Science and Technology, the Ministry of International Trade and Industry)(1-1-3 Higashi, Tsukuba-shi, Ibaraki 305-8566, Japan) under the Accession Number FERM BP-7403 since December 21, 2000; and with Institute for Fermentation (IFO)(2-17-85 Juso Honcho, Yodogawa-ku, Osaka-shi, Osaka 532-8686, Japan) under the Accession Number IFO 16512 since December 6, 2000.

The transformant *Escherichia coli* XL1-Blue/pTB2187 obtained in Example 2 is on deposit with International Patent Organism Depositary, National Institute of Advanced Industrial Science and Technology (the successor of National Institute of Bioscience and Human Technology (NIBH), Agency of Industrial Science and Technology, the Ministry of International Trade and Industry)(1-1-3 Higashi, Tsukuba-shi, Ibaraki 305-8566, Japan) under the Accession Number FERM BP-7404 since December 21, 2000; and with Institute for Fermentation (IFO)(2-17-85 Juso Honcho, Yodogawa-ku, Osaka-shi, Osaka 532-8686, Japan) under the Accession Number IFO 16513 since December 6, 2000.

The transformant *Escherichia coli* TOP10/pTB2198 obtained in Example 2 is on deposit with International Patent Organism Depositary, National Institute of Advanced Industrial Science and Technology (the successor of National Institute of Bioscience and Human Technology (NIBH), Agency of Industrial Science and Technology, the Ministry of International Trade and Industry)(1-1-3 Higashi, Tsukuba-shi, Ibaraki 305-8566, Japan) under the Accession Number FERM BP-7424 since January 11, 2001; and with Institute for Fermentation (IFO)(2-17-85 Juso Honcho, Yodogawa-ku, Osaka-shi, Osaka 532-8686, Japan) under the Accession Number IFO 16519 since December 22, 2000.

### EXAMPLES

The present invention is described in more detail with reference to the following examples, but not intended to limit the scope of the present invention thereto. The genetic procedures using *Escherichia coli* were performed according to methods described in the "Molecular Cloning".

### Example 1

Using primers shown below, PCR was carried out in which various human cDNAs were used as the template (5 cycles of reactions at 94°C for 10 seconds, at 60°C for 30 seconds and at 72°C for 1 minute and then 30 cycles of reactions at 94°C for 10 seconds, at 55°C for 30 seconds and at 72°C for 1 minute).

Primer 1: 5'-GGAGACCAGACCGAACTATTG-3' [SEQ ID NO:5]

Primer 2: 5'-TGAAGAGATGGTGATGCTACAGG-3'          [SEQ ID NO:6]

As a result, it was revealed that a gene highly homologous to human-derived PSMA (prostate-specific membrane antigen) is contained in several kinds of cDNA libraries. To obtain its full-length gene, the full-length sequence was determined by an RACE (rapid amplification of cDNA ends) method.

First, primers shown below were designed on the basis of the sequence found above, and PCR was carried out by using the primers and AP1 and AP2 primers attached to Marathon cDNA Amplification Kit manufactured by CLONTECH, together with human i prostate-derived Marathon ready cDNA (CLONTECH) as the template, and thereby further cloning of its 5' upstream fragment was attempted. The PCR conditions were those in accordance with a protocol attached to the kit.

Primer 3: 5'-TGAAGAGATGGTGATGCTACAGGATACAGA-3'     [SEQ ID NO:7]

Primer 4: 5'-GCCAATATTGCCAAAAGCTGTTCCTC-3' [SEQ ID NO:8]

The resulting PCR fragment contained a sequence considered to be a translation initiation codon.

On the other hand, primers shown below were designed for cloning its 3' downstream region, and PCR was carried out according to the method described above. The primers used are as follows:

Primer 5: 5'-GTTGGCAGCCATCATCACACTGCACACAGT-3'     [SEQ ID NO:9]

Primer 6: 5'-GAGGAACAGCTTTTGGCAATATTGGC-3' [SEQ ID NO:10]

The PCR fragment thus obtained was composed of two PCR fragments having base substitution at one site, and the base substitution involved amino acid substitution. Further, the two PCR fragments contained a sequence considered to be a translation termination codon.

The gene fragments obtained thus by the RACE method were ligated by a general method described in "Molecular Cloning" etc., to give a full-length fragment of about 3.6 kb. This full-length gene sequence contained an ORF sequence consisting of 2385 bp, and it was revealed as shown in the Sequence Listing that in the nucleotide sequence set forth in SEQ ID NO:2, the base at the 2030 position is T (thymine), and in the nucleotide sequence set forth in SEQ ID NO:4, the base at the 2030 position is C (cytosine).

The amino acid sequences (SEQ ID NOS:1 and 3) deduced respectively from SEQ ID NOS:2 and 4 had 26% homology with PSMA reported as human carboxy peptidase protein, 24% homology with rat dipeptidyl aminopeptidase I-100, 21% homology with human transferrin receptor, and 22% homology with human transferrin receptor 2α, thus suggesting that they are novel peptidase proteins also functioning as receptors.

### Example 2

After the following primers containing a translation initiation codon and a translation termination codon respectively were designed on the basis of the ORF sequence obtained in Example 1, PCR was conducted by using human prostate-derived Marathon ready cDNA (CLONTECH) as the template.

Primer 7: 5'-GCTCGAGATGGGAGAGAATGAAGCAAGTTTACCTAACACGTC-3'
[SEQ ID NO:11]

Primer 8: 5'-GGAATTCTCAATTCTTCCCATCCAAGACACTCTTGAACAC-3'
[SEQ ID NO:12]

The reaction solution in this reaction, adjusted to a volume of 100 µl, was composed of 20 µl of the above cDNA as the template, 5 U of Pfu Turbo DNA polymerase (Stratagene), 0.4 µM each of primers 7 and 8, 400 µM dNTPs, and 50 µl of 2xGC Buffer I (Takara Shuzo Co., Ltd.). The PCR consisted of a reaction at 94°C for 1 minute, then 35 cycles each consisting of reactions at 94°C for 10 seconds, at 55°C for 30 seconds and 72°C for 3 minutes and then an elongation reaction at 72°C for 7 minutes. A part of the PCR product was cloned directly in plasmid vector pCR-Blunt (Invitrogen) according to a protocol of a Zero Blunt PCR cloning kit (Invitrogen), while 2.5 U of Ex-Taq (Takara Shuzo) was added to the remainder of the PCR product, and the mixture was reacted at 72°C for 10 minutes and then subcloned in plasmid vector pCRII-TOPO (Invitrogen) according to a protocol of a TOPO TA cloning kit (Invitrogen). These resulting vectors were introduced into E. coli DH5α, XL1-Blue and TOP10, and clones having the cDNA were selected in an LB agar medium containing kanamycin or ampicillin. As a result of analysis of the respective clones, the two cDNA sequences (SEQ ID NO:2 and 4) described in Example 1 were obtained from the cDNA sequences subcloned in the vector pCR-Blunt, while a new cDNA sequence (SEQ ID NO:14) encoding a novel peptidase protein (SEQ ID NO:13) was obtained from the cDNA sequences subcloned in the vector pCRII-TOPO. Plasmids containing the cDNAs set forth in SEQ ID NOS:2, 4 and 14 were designated pTB2187, pTB2186 and pTB2198. Their respective transformants were designated Escherichia coli XL1-Blue/pTB2187, DH5α/pTB2186, and TOP10/pTB2198, respectively.

### Example 3 Construction of E. coli expression vector

Expression of a recombinant protein of a part corresponding to the extracellular region of the protein of this invention was carried out in E. coli. That is, PCR was carried out in which pTB2186 obtained in Example 2 was used as a template, together with the following primers:

Primer 9: 5'-ATGAGTGAAGAAGCCAGAAAAGATAGCAG-3' [SEQ ID NO:15]

Primer 10: 5'-ATTCTTCCCATCCAAGACACTCTTGAACAC-3'    [SEQ ID NO:16]

The reaction solution in this reaction, adjusted to a volume of 100 µl, was composed of 5 ng of the above plasmid as the template, 5 U of Pfu Turbo DNA polymerase (Stratagene), 0.4 µM each of primers 9 and 10, 400 µM dNTPs, and 50 µl of 2xGC Buffer I (Takara Shuzo). The PCR consisted of a reaction at 94°C for 1 minute, then 20 cycles each consisting of reactions at 94°C for 10 seconds, at 55°C for 30 seconds and 72°C for 2 minutes, and then an elongation reaction at 72°C for 5 minutes. 2.5 U of Ex-Taq (Takara Shuzo) was added to the PCR reaction product, then the mixture was reacted at 72°C for 10 minutes, and the PCR product was purified by a MinElute PCR Purification kit (QIAGEN). This product was inserted into plasmid vector pCRT7/CT-TOPO (Invitrogen) according to a protocol of a pCRT7/CT TOPO TA cloning kit (Invitrogen) and then used to transform E. coli TOP10F. Clones carrying a plasmid containing the PCR amplification product were selected from the E. coli, and the sequences of plasmids extracted from the respective clones were analyzed, and a plasmid (pREC) with no error in the base sequence was obtained.

### Example 4 Expression in E. coli and purification of the recombinant protein

E. coli BL21(DE3) pLys S was transformed with the plasmid pREC obtained in Example 3, and then used for the expression. Induction of the expression was carried out with 0.5 mM isopropyl thiogalactopyranoside, and purification was carried out by using ProBond (Invitrogen) according to its attached manual. As a result, the desired recombinant protein of about 65 kD was eluted with Denaturating Elution Buffer (ProBond Purification System, Invitrogen). Then, the recombinant protein, in a dialysis membrane for fractionation molecular weights of 6000 to 8000 (SPECTRUM MEDICAL), was dialyzed against a buffer [20 mM Tris-HCl (pH 7.4), 0.5 M L-arginine] at 4°C. The recombinant protein, 4.3 mg, could be obtained from 1 L culture solution.

### Example 5 Preparation and purification of rabbit polyclonal antibody

A rabbit polyclonal antibody was prepared using the recombinant protein prepared in Example 4. An animal to be immunized was one male rabbit KBL:JW (10-week-old, Oriental Yeast Co., Ltd.) and sensitized by injecting the protein subcutaneously into the back 3 times at 14-day intervals. The amount of the recombinant protein used in each sensitization was 0.5 mg, and a complete Freund's adjuvant (Difco) suspension was used. At 38 days after the first sensitization, blood was collected through a carotid artery from the animal under anesthesia, to give about 55 ml serum. The whole of the serum thus obtained was concentrated by salting-out with sulfate ammonium, and the whole of the resulting crude IgG fraction was purified through a Protein A affinity column (Amersham-Pharmacia), whereby about 410 mg of the purified IgG fraction was obtained as a polyclonal antibody (AS-2157) fraction.

### Example 6 Preparation and purification of peptide antibodies

On the basis of the amino acid sequence set forth in SEQ ID NO:1, the following 2 partial peptides each consisting of 15 amino acids were synthesized:
Peptide 1: Lys-Leu-Ile-Ser-Ser-Pro-Lys-Ala-Arg-Thr-Lys-Asn-Glu-Ala-Cys [SEQ ID NO:17]
Peptide 2: Ser-Asp-Glu-Met-Arg-Pro-Ala-Asn-Asp-Pro-Lys-Glu-Arg-Ala-Cys [SEQ ID NO: 18]

Each peptide was used as an antigen after binding it to keyhole limpet hemocyanin (KLH) as a carrier protein. Sensitization with the antigen was carried out according to the method described in Example 5 above. 0.5 mg antigen was used in each sensitization, and at 52 days after the first sensitization, whole blood was collected, and about 70 ml serum was obtained for each antigen. The whole serum was subjected to the same procedure as in Example 5 to give 665 mg and 444 mg purified IgG fractions respectively for Peptides 1 and 2. The two fractions, 200 mg and 138 mg respectively, were purified by columns having each peptide immobilized thereon. For immobilization, each peptide was coupled via its C-terminal cysteine with a Sepharose column (Amersham-Pharmacia) in a borate buffer. For elution from the column, 8M urea/phosphate buffered physiological saline (PBS) was used. The eluate was dialyzed against PBS to remove urea, then concentrated by ultrafiltration, and sterilized by filtration to give about 7 mg affinity-purified antibodies AS-1988 and 1989 for Peptides 1 and 2, respectively.

### Example 7 Construction of an expression vector for animal cells

An expression vector was constructed to express a protein having the amino acid sequence set forth in SEQ ID NO:I in animal cells. That is, PCR was conduced in which pTB2186 obtained in Example 2 was used as the template, together with the following primers:

Primer 10: 5'-ATTCTTCCCATCCAAGACACTCTTGAACAC-3'     [SEQ ID NO:16]

Primer 11: 5'-ATGGGAGAGAATGAAGCAAGTTTACCTAAC-3'   [SEQ ID NO:19]

The reaction solution in this reaction, adjusted to a volume of 50 µl, was-composed of 2.5 ng of the above plasmid as the template, 2.5 U of Pfu Turbo DNA polymerase (Stratagene), 0.4 µM each of primers 10 and 11, 400 µM dNTPs, and 25 µl of 2×GC Buffer I (Takara Shuzo). The PCR consisted of a reaction at 94°C for 1 minute, then 20 cycles each consisting of reactions at 94°C for 15 seconds, at 55°C for 30 seconds and at 72°C for 2.5 minutes, and then an elongation reaction at 72°C for 7 minutes. 2.5 U of Ex-Taq (Takara Shuzo) was added to the PCR reaction product, then the mixture was reacted at 72°C for 10 minutes, and the PCR product was purified by a MinElute PCR Purification kit (QIAGEN). This product was inserted into plasmid vector pcDNA3.1/V5-His-TOPO (Invitrogen) according to a protocol of a pcDNA3.1/V5-His TOPO TA cloning kit (Invitrogen) and then used to transform E. coli TOP 10. Clones carrying a plasmid containing the PCR amplification product were selected from the E. coli, and the sequences of plasmids extracted from the respective clones were analyzed, and a plasmid (pcDNA-REC) with no error in the base sequence was obtained.

### Example 8 Western blotting using the peptide antibodies

Detection of a protein having the amino acid sequence set forth in SEQ ID NO: 1 was conducted using the peptide antibodies prepared in Example 6. Fibroblast COS7 cells (8×10⁵) derived from monkey kidney were suspended in 10 ml Dulbecco's modified Eagle minimum medium (Invitrogen) containing 10% fetal bovine serum (Invitrogen) and then inoculated into a Petri dish of 10 cm in diameter. Then, the cells were cultured at 37°C overnight under 5% carbon dioxide atmosphere, and then 6 µg pcDNA-REC previously mixed with 18 µl FuGENE6 transfection reagent (Roche Diagnosis) and left at room temperature for 15 minutes was added to the cells which were then cultured under the same conditions. After 2 days, the cells were washed with PBS, and 1 ml ice-cooled RIPA buffer (50 mM Tris-HCl, pH 7.5, 0.15 M sodium chloride, Complete™ tablet (Roche Diagnosis), 1% Triton X-100, 0.1% sodium dodecyl sulfate) was added to the cells and left at 4°C for 15 minutes. After recovery of the RIPA buffer and centrifugation at 10000×g for 20 minutes, 10 µl supernatant as a cell-free extract was subjected to SDS-PAGE on 10% acrylamide gel. The separated proteins were transferred in a usual manner onto Clear Blot Membrane P (ATTO) and then left for 1 hour at room temperature in a blocking solution (Tris buffered physiological saline, 0.1% Tween 20, 5% skimmed milk). Then, the membrane was incubated at 4°C overnight in the blocking solutions into which the peptide antibodies AS-1988 and 1989 prepared in Example 6 had been added at a concentration of 5 µg/ml respectively, and then left for 1 hour at room temperature in a secondary antibody solution prepared by diluting an anti-rabbit IgG-HRP conjugate (Amersham-Pharmacia) 100,000-fold with the blocking solution. Detection was carried out by using ECL plus (Amersham-Pharmacia) according to its attached manual. Whichever the peptide antibody AS-1988 or 1989 was used, a specific band derived from the protein of this invention was recognized at a position in the vicinity of the molecular weight of 100 kD in Precision prestained marker (Bio-Rad).

### Example 9 Examination of the gene expression level in human non-small cell lung cancer tissues

Using cDNA (Biochain) derived from human lung cancer tissues and first-strand cDNA (Clontech) derived from normal human lung tissues were used as the template, PCR was carried out with a pair of primers shown below thereby comparing the expression level of the gene encoding the protein of this invention in the cancer tissues with that in the normal tissues.

Primer 12: 5'-GCTCGAGATGGGAGAGAATGAAGCAAGTTTACCTAACACGTC-3'
[SEQ ID NO:20]

Primer 13: 5'-GGAATTCTCAATTCTTCCCATCCAAGACACTCTTGAACAC-3'
[SEQ ID NO:21]

The reaction solution in this reaction, adjusted to a volume of 20 µl, was composed of 1 µl of the above cDNA as the template diluted at various concentrations, 1.5 U of La-Taq DNA polymerase (Takara Shuzo), 0.4 µM each of primers 12 and 13, 400 µM dNTPs, 2.5 mM MgCl₂ and 2 µl of 10×La-Taq Buffer II (Mg²⁺ free, Takara Shuzo). The PCR consisted of a reaction at 94°C for 1 minute, then 40 cycles each consisting of reactions at 94°C for 10 seconds, at 65°C for 30 seconds and at 72°C for 2.5 minutes. As the negative control, the expression level of β-actin gene was examined at the same time. That is, PCR amplification was carried out after replacing the primers in the above reaction solution with a commercially available human actin gene primer set (Clontech). The PCR consisted of a reaction at 94°C for 1 minute, then 20 cycles each consisting of reactions at 94°C for 30 seconds, at 60°C for 30 seconds and 72°C for 1 minute, and then an elongation reaction at 72°C for 5 minutes. As a result of correction based on the expression level of the actin gene, it was revealed that the expression of the gene encoding the protein of this invention in the human cancer lung tissues is promoted about 30 times as compared with that in the normal tissues.

### Example 10 Examination of the gene expression level in human prostate cancer tissues

PCR was carried out using a tumor/normal matched cDNA pair (Clontech) derived from human prostate cancer tissues as the template, together with a pair of primers shown below and an FAM-labeled TaqMan probe, to compare the expression level of the gene encoding the protein of this invention in cancer tissues with that in normal tissues.

Primer 14: 5'-TCAACCATCTTGGAGTTCCCA-3'          [SEQ ID NO:22]

Primer 15: 5'-CGGAGAGAAAACTTGGACCCTC-3'     [SEQ ID NO:23]

TaqMan probe: 5'-CGTGCAGTTTGCTTACGAGGACATCAAA-3'[SEQ ID NO:24]

The reaction solution in this reaction, adjusted to a volume of 25 µl, was composed of 1 µl of the above cDNA as the template, 12.5 µl TaqMan universal PCR master mix (Applied Biosystems), 0.4 µM each of primers 14 and 15, and 200 nM TaqMan probe. The PCR consisted of reactions at 50°C for 2 minutes and at 95°C for 10 minutes and then 40 cycles each consisting of reactions at 95°C for 15 seconds and at 60°C for 1 minute. As a result, it was revealed that the expression of the gene encoding the protein of this invention in the human prostate cancer tissues is promoted 14 times as compared with that in the surrounding normal tissues.

### Example 11 Examination of the gene expression level in human cancer cell strains

Since it was revealed in Examples 9 and 10 that an increase in the expression level of the gene encoding the protein of this invention was recognized in human lung cancer tissues and prostate cancer tissues, the same examination was also carried out in cultured cell strains. Human lung cancer cell strains NCI-H2342, NCI-H1435, NCI-H1581, human prostate cancer cell strain LNCaP (each of which was purchased from ATCC), and human normal small respiratory tract epithelial cells SAEC and human normal prostate epithelial cells PrEC (each of which was purchased from Clonetics)( each 10⁶ to 10⁷ cells) were recovered and used to prepare total RNA by using an RNeasy mini kit (QIAGEN). By RT-PCR using this total RNA as the template together with the primer set and the TaqMan probe used in Example 10, the gene expression level was calculated. The reaction solution in this reaction, adjusted to a volume of 25 µl, was composed of 1 ng of the total RNA as the template, 12.5 µl of TaqMan one-step RT-PCR master mix (Applied Biosystems), 0.625 µl of MultiScribe & RNase inhibitor mix (Applied Biosystems), 0.4 µM each of primers 14 and 15, and 200 nM TaqMan probe. The PCR consisted of a reactions at 48°C for 30 minutes and at 95°C for 10 minutes and then 40 cycles each consisting of reactions at 95°C for 15 seconds and at 60°C for 1 minute. Simultaneously, TaqMan human β-actin control reagent (Applied Biosystems) was used to calculate the number of copies of β-actin gene contained in 1 ng total RNA as the internal standard. The reaction composition and PCR conditions were in accordance with those described above. To eliminate the effect of contaminated genomic DNA, the same procedure was carried out without using the MultiScribe & RNase inhibitor mix, and the determined number of copies was subtracted. As a result, the expression level in SAEC and PrEC cells was 0.1% and 0.03% of the expression level of β-actin gene, respectively, while the expression levels in the human lung cancer cell strains NCI-H2342, NCI-H1435 and NCI-H1581 were 2.6 %, 1.1% and 1.2%, respectively, and the expression level in the LNCaP cells was 5.7%, thus indicating a significant increase in the expression.

### Example 12 Immunofluorescence staining of LNCaP cells with the peptide antibody

Since the expression of the protein of this invention in the LNCaP cells was revealed in Example 11, immunostaining of the LNCaP cells with the peptide antibody AS-1988 obtained in Example 6 was carried out. LNCaP cells (10⁵) were suspended in Dulbecco's modified Eagle minimum medium containing 10% fetal bovine serum and inoculated in a 4-well Lab-Tek chamber (Nunc) and cultured at 37°C for 1 day under 5% carbon dioxide atmosphere. On the next day, the medium was exchanged with a fresh medium not containing the serum, and the cells were further cultured overnight. The medium was removed, and after addition of 2% p-formaldehyde/PBS, the cells were fixed at room temperature for 15 minutes and then washed PBS, and after exchange with 0.2% Triton X-100/PBS, the cells were incubated at room temperature for 5 minutes, then washed with PBS and blocked with 5% bovine serum albumin/PBS (blocking solution) at room temperature for 30 minutes. The cells were incubated at 4°C overnight with AS-1988 previously diluted at 5 µg/ml with the blocking solution, then washed with PBS, and incubated at 4°C for 3 hours with 1 µg/ml Cy3-labeled mouse anti-rabbit IgG antibody in the blocking solution. The cells were washed with PBS and observed under a fluorescence microscope, and as a result, a fluorescent image stained strongly in the edge of the cells was recognized, suggesting that the protein of this invention is localized on the cytoplasmic membrane.

### Example 13 Examination of localization by centrifugal fractionation of cells

The method of fractionating cells by ultracentrifugation was in accordance with an already reported method (Experimental Note in Protein Experiment (in Japanese), volume I, pp. 65-66, Yodosha, 1996). Human prostate cancer cell strain LNCaP was cultured in a Petri dish of 10 cm in diameter in the same manner as in Example 12 and after the medium was exchanged with a serum-free medium, the cells were cultured overnight and then suspended in 2 ml basal buffer (50 mM Tris-HCI, pH 7.4, Complete™ tablet) containing 0.25 M sucrose under cooling on ice. The cells were disrupted with a Dounce homogenizer (Wheaton) and centrifuged at 1000×g for 7 minutes to remove undisrupted cells, and then centrifuged at 1600×g for 20 minutes to recover a precipitated fraction. This fraction was suspended in 2 ml of the same buffer containing 0.25 M sucrose and layered on 10 ml basal buffer containing 1.25 M sucrose. After centrifugation at 116,000×g for 1 hour, the protein concentrated in the boundary between the 2 layers was recovered with a Pasteur pipette and diluted to a 5-fold volume with 10 mM Tris-HCl (pH 7.4). This dilution was centrifuged at 42000xg for 20 minutes, to recover precipitates of the cytoplasmic membrane fraction. When the recovered protein was detected by Western blotting with the antibody AS-1989 in the same manner as in Example 7, a band was detected at a position of the molecular weight of the protein of this invention, revealing that the protein of this invention is localized on the cytoplasmic membrane. On the other hand, when the LNCaP cells were cultured in the same manner as above in the presence of 10% fetal bovine serum until the cell were recovered, the localization of the protein of this invention in the cytoplasmic membrane was not recognized, thus suggesting that a factor in serum induces the intracellular localization of the protein of this invention, and thus that the protein of this invention functions as a receptor.

### Example 14 Measurement of tyrosine phosphorylation in the protein of this invention

Fibroblast COS7 cells (8.7×10⁵) derived from monkey kidney were suspended in 10 ml Dulbecco's modified Eagle minimum medium (Invitrogen) containing 10% fetal bovine serum (Invitrogen) and inoculated in a Petri dish of 10 cm in diameter. The cells were cultured at 37°C overnight under 5% carbon dioxide atmosphere, and then 6 µg pcDNA-REC previously mixed with 18 µl FuGENE6 transfection reagent (Roche Diagnosis) and left at room temperature for 15 minutes were added to the cells which was then cultured under the same conditions. After 2 days, the cells were washed 3 times with PBS containing 1 mM sodium o-vanadate, and then I ml ice-cooled RIPA buffer (50 mM Tris-HCl, pH 7.5, 0.15 M sodium chloride, Complete™ tablet (Roche Diagnosis), 1% Triton X-100, 0.1% sodium dodecyl sulfate) containing 1 mM sodium o-vanadate was added to the cells which were then left at 4°C for 15 minutes. The RIPA buffer was recovered, and the cell were centrifuged at 10000×g for 20 minutes thereby separating a supernatant as a cell-free extract. Anti-V5 tag antibody (Invitrogen) was added at a concentration of 1 µg/ml to the cell-free extract, and 50 µl of Protein G Sepharose (Amersham-Pharmacia) was added thereto and stirred at 4°C overnight. By centrifugation at 10,000×g at 4°C for 1 minute, a fraction bound to the Protein G Sepharose was recovered and washed 3 times with ice-cooled RIPA buffer. The Protein G Sepharose fraction separated by centrifugation under the above-mentioned conditions was suspended in 50 µl Laemmli buffer, and its aliquot of 20 µl was subjected to SDS-PAGE on 10% acrylamide gel. The protein was transferred in a usual manner onto Clear Blot Membrane P (ATTO) and left for 2 hours in a blocking solution (Tris-buffered physiological saline, 0.1% Tween-20, 1% bovine serum albumin) at room temperature. Detection was carried out by using a phosphotyrosine detection kit (Amersham-Pharmacia) and ECL plus (Amersham-Pharmacia) according to their attached manuals. As a result, a band was recognized at a position of the molecular weight of the protein of this invention, indicating that a tyrosine residue in the protein of this invention is phosphorylated in culture in the presence of fetal bovine serum.

### Example 15 Preparation and evaluation of immunotoxins

For the purpose of creation of pharmaceutical preparations of antibodies to human cancer cells highly expressing the protein of this invention, the antibody AS-2157, AS-1988 or AS-1989 prepared in Example 5 or 6 was used to prepare immunotoxins. The preparation method followed a method of Chari, R. V. J. et al. (Cancer Res. 52:127-131 (1992); US Patent 5,208,020 (1993)). That is, 7.5 mg or 1 mg of rabbit IgG purified by a Protein A column or a peptide-immobilized column was used as the starting material. N-succinimidyl-3-(2-pyridyldithio)propionate was used to introduce a dithiopyridyl group into the antibody, and a maytansine derivative was attached via S-S-crosslinkage to the antibody, to form an immunotoxin. As an immunotoxin for negative control, 7.5 mg ChromPure™ non-immunized rabbit IgG (Jackson ImmunoResearch) was used to prepare a conjugate with a maytansine derivative in the same manner. The number of maytansine derivative molecules bound to one immunotoxin molecule thus obtained was revealed to be 3.3 to 3.7 by calculation based on absorbance at 280 nm and 252 nm. When the immunotoxin thus prepared was examined for its cytotoxic activity on the human lung cancer cell strain NCI-H2342 found in Example 11, a growth inhibitory activity specific to the antibody was recognized. That is, the minimum effective concentration [concentration for 30% growth inhibition] of the negative control immunotoxin was higher than 67 nM, while the minimum effective concentration of the AS-2157-derived immunotoxin was 3.3 nM, and that of the AS-1989-derived immunotoxin was 37 nM, indicating that the protein of this invention and partial peptides thereof are useful as the antigen for anti-tumor immunotoxins.

### INDUSTRIAL APPLICABILITY

The proteins of the present invention and the DNAs encoding the same can be used as a therapeutic and/or prophylactic agent for diseases such as cancers and neurological diseases. In addition, the proteins of the present invention or the cells capable of expressing the genes of the proteins are useful as a reagent for the screening of a compound capable of enhancing or inhibiting the peptidase activity of the proteins of the present invention. Further, the antibodies to the proteins of the present invention can recognize specifically to the proteins of the present invention, and thus can be used for the quantification of the proteins of the present invention contained in a liquid sample.

## Claims

1. A protein comprising the same or substantially the same amino acid sequence as that shown by SEQ ID NO: 1 or a salt thereof.

2. The protein described in claim 1 comprising the amino acid sequence shown by SEQ ID NO: 1, NO: 3 or NO: 13 or a salt thereof.

3. A partial peptide of the protein described in claim 1 or a salt thereof.

4. A polynucleotide comprising a polynucleotide encoding the protein described in claim 1 or the partial peptide described in claim 3.

5. The polynucleotide described in claim 4 which is a DNA.

6. The DNA described in claim 5 comprising the nucleic acid sequence shown by SEQ ID NO: 2, NO: 4 or NO: 14.

7. A recombinant vector comprising the polynucleotide described in claim 4.

8. A transformant, which is transformed with the recombinant vector described in claim 7.

9. A method of producing the protein described in claim 1 or a salt thereof, which comprises culturing the transformant described in claim 8 to produce and accumulate the protein described in claim 1; and recovering it.

10. A pharmaceutical composition comprising the protein described in claim 1, the partial peptide described in claim 3, or a salt thereof.

11. A pharmaceutical composition comprising the polynucleotide described in claim 4.

12. The pharmaceutical composition described in claim 10 or 11, which is a therapeutic and/or prophylactic agent for cancers or neurological disorders.

13. An antibody to the protein described in claim 1, the partial peptide described in claim 3 or a salt thereof.

14. A diagnostic agent for cancers or neurological disorders, comprising the antibody described in claim 13.

15. A complex comprising the antibody described in claim 13 and a toxin.

16. A method of screening a compound or a salt thereof capable of enhancing or inhibiting the peptidase activity of the protein described in claim 1, the partial peptide described in claim 3 or a salt thereof, which comprises using the protein described in claim 1, the partial peptide described in claim 3 or a salt thereof.

17. A kit for screening a compound or a salt thereof capable of enhancing or inhibiting the peptidase activity of the protein described in claim 1, the partial peptide described in claim 3 or a salt thereof, which comprises the protein described in claim 1, the partial peptide described in claim 3 or a salt thereof.

18. A compound or a salt thereof capable of enhancing or inhibiting the peptidase activity of the protein described in claim 1, the partial peptide described in claim 3 or a salt thereof, which is obtained using the screening method described in claim 16 or the screening kit described in claim 17.

19. A pharmaceutical composition comprising a compound or a salt thereof capable of enhancing or inhibiting the peptidase activity of the protein described in claim 1, the partial peptide described in claim 3 or a salt thereof, which is obtained using the screening method described in claim 16 or the screening kit described in claim 17.

20. The pharmaceutical composition described in claim 19, which is a therapeutic and/or prophylactic agent for cancers or neurological disorders.

21. A pharmaceutical composition comprising the antibody described in claim 13 or the complex described in claim 15.

22. The pharmaceutical composition described in claim 21, which is a therapeutic and/or prophylactic agent for cancers.

23. An antisense polynucleotide comprising a nucleic acid sequence which is complementary or substantially complementary to a polynucleotide encoding a protein having the same or substantially the same amino acid sequence as that shown by SEQ ID NO: 1 or a partial peptide thereof.

24. A pharmaceutical composition comprising the antisense polynucleotide described in claim 23.

25. The pharmaceutical composition described in claim 24, which is a therapeutic and/or prophylactic agent for cancers or neurological disorders.

26. Use of the protein described in claim 1 or a salt thereof, the partial peptide described in claim 3 or a salt thereof, the polynucleotide described in claim 4, the antibody described in claim 13, the complex described in claim 15, the compound described in claim 18 or a salt thereof, or the antisense polynucleotide described in claim 23 for producing a pharmaceutical composition for the treatment and/or prevention of cancers or neurological disorders.

27. A method of treating and/or preventing cancers or neurological disorders in a human or another mammal, which comprises administering to said human or mammal an therapeutically or prophylactically effective amount of the protein described in claim 1 or a salt thereof, the partial peptide described in claim 3 or a salt thereof, the polynucleotide described in claim 4, the antibody described in claim 13, the complex described in claim 15, the compound described in claim 18 or a salt thereof, or the antisense polynucleotide described in claim 23.
